# EUROPÄISCHE PATENTSCHRIFT

(11) **EP 2 940 143 B1**
(45) Veröffentlichungstag und Bekanntmachung des Hinweises auf die Patenterteilung: **05.02.2020**
(21) Anmeldenummer: 14166633.9
(22) Anmeldetag: 30.04.2014
(51) Int. Cl.: C12P 13/04, C12N 9/06, C12N 9/78, C12N 9/86, C12N 9/00, C12N 15/63

(54) **Verfahren zur Produktion von L-Aminosäuren unter Verwendung eines alkaliphilen Bakteriums**
Method for the production of l-amino acids using an alkaliphilic bacterium
Procédé de production d'acides L-aminés en utilisant une bactérie alcaliphile

(43) Veröffentlichungstag der Anmeldung: 04.11.2015
(73) Patentinhaber: Evonik Operations GmbH, 45128 Essen (DE)
(72) Erfinder: Ochrombel, Dr. Ines, 33729 Bielefeld (DE); Bathe, Dr. Brigitte, 33154 Salzkotten (DE); Hasselmeyer, Marleen, 33649 Bielefeld (DE); Kalinowski, Prof. Jörn, 33615 Bielefeld (DE); Rückert, Christian, 33335 Gütersloh (DE); Persicke, Dr. Marcus, 33739 Bielefeld (DE)
(74) Vertreter: Evonik Patent Association

(56) Entgegenhaltungen:
- EP-A2- 1 108 790
- CN-A- 101 892 180
- SAHM HERMANN ET AL: "Metabolic design in amino acid producing bacterium Corynebacterium glutamicum", FEMS MICROBIOLOGY REVIEWS, Bd. 16, Nr. 2-3, 1995, Seiten 243-252, XP002730850, ISSN: 0168-6445
- BARRIUSO-IGLESIAS MONICA ET AL: "Transcriptional analysis of the F0F1 ATPase operon of Corynebacterium glutamicum ATCC 13032 reveals strong induction by alkaline pH", MICROBIOLOGY (READING), Bd. 152, Nr. Part 1, Januar 2006 (2006-01) , Seiten 11-21, XP002730851, ISSN: 1350-0872
- FERMENTATION PRODUCTION OF L-HOMOSERINE BY CORYNEBACTERIUM SP AND ITS POSSIBLE USE IN THE PREPARATION OF THREONINE AND LYSINE: "Fermentation production of L-Homoserine by Corynehacterium sp. and its possible use in the preparation of threonine and lysine", FOLIA MICROBIOLOGICA, PRAQUE, CZ, Bd. 30, Nr. 6, 1. November 1985 (1985-11-01), Seiten 485-492, XP008144841, ISSN: 0015-5632, DOI: 10.1007/BF02927611
- IKEDA KOJI ET AL: "Isolation and characterization of a novel facultatively alkaliphilic bacterium, Corynebacterium sp., grown on n-alkanes", ARCHIVES OF MICROBIOLOGY, Bd. 162, Nr. 6, 1994, Seiten 381-386, XP008172588, ISSN: 0302-8933
- WU CHUN-YUAN ET AL: "Corynebacterium humireducens sp. nov., an alkaliphilic, humic acid-reducing bacterium isolated from a microbial fuel cell", INTERNATIONAL JOURNAL OF SYSTEMATIC AND EVOLUTIONARY MICROBIOLOGY, Bd. 61, Nr. 4, April 2011 (2011-04), Seiten 882-887, XP002692942, ISSN: 1466-5026, DOI: 10.1099/ijs.0.020909-0
- Christian Rückert ET AL: "Genome sequence of the halotolerant bacterium Corynebacterium halotolerans type strain YIM 70093T (= DSM 44683T); extract", Standards in Genomic Sciences, 1 December 2012 (2012-12-01), pages 284-293, XP55531176, DOI: 10.4056/sigs.3236691 Retrieved from the Internet: URL:https://www.ncbi.nlm.nih.gov/nuccore/C P003697

## Beschreibung

Die vorliegende Erfindung betrifft ein Verfahren zur Produktion von L-Aminosäuren, in welchem ein alkaliphiles Bakterium, insbesondere ein Stamm der Spezies Corynebacterium humireducens, verwendet wird.

Verfahren zur Produktion von L-Aminosäuren, in welchen Bakterien der Gattung Corynebacterium eingesetzt werden, sind dem Fachmann bekannt.

Obwohl zahlreiche Corynebacterium-Arten bekannt sind, werden in diesen Verfahren üblicherweise Bakterien der Art Corynebacterium glutamicum eingesetzt, da sich diese Art als besonders vorteilhaft für die Produktion von L-Aminosäuren herausgestellt hat.

So wird in EP 1 108 790 A2 ein Verfahren zur Herstellung von L-Lysin in coryneformen Bakterien, insbesondere in Corynebacterium glutamicum, beschrieben.

Sahm et al. (1995) "Metabolic design in amino acid producing bacterium Corynebacterium glutamicum", FEMS Microbiology Reviews, Bd. 16, Nr. 2-3, S. 243-252, offenbaren rekombinante Stämme von C. glutamicum, die für die Produktion verschiedener Aminosäuren verwendet werden können.

Monica Barriuso-Iglesias et al. (2006), Microbiology (Reading), Bd. 152, Part 1, S. 11-21, beschreiben die transkriptionale Analyse des F0F1-ATPase-Operons von C. glutamicum ATCC 13032.

Plachy et al. (1985), Folia Microbiologica, Praque, CZ, Bd. 30, Nr. 6, S. 485-492, beschreiben, dass Corynebacterium sp. für die fermentative Produktion von L-Homoserin, L-Lysin und L-Threonin verwendet werden kann.

Ikeda et al. (1994), Archives of Microbiology, Bd. 162, Nr. 6, S. 381-386, beschreiben die Isolation und Charakterisierung eines neuen, fakultativ alkaliphilen Bakterium der Spezies Corynebacterium.

Wu et al. (2011), International Journal of Systematic and Evolutionary Microbiology, Bd. 61, Nr.4, S. 882-887, sowie CN 101 892 180 A berichten beide über die Isolierung und biochemische Charakterisierung von Corynebacterium humireducens.

Aufgabe der vorliegenden Erfindung war es, einen neuen Stamm zur Verfügung zu stellen, der als Alternative zu C. glutamicum entweder unmittelbar für die Produktion von L-Aminosäuren in Frage kommt, weil er eine signifikante Überproduktion an mindestens einer L-Aminosäure aufweist, oder aber zumindest als erfolgversprechender Ausgangsstamm für die Entwicklung eines neuen L-Aminosäuren-Produktionsstamms in Betracht kommt.

Um als Ausgangsstamm für die Entwicklung eines neuen L-Aminosäuren-Produktionsstamms in Frage zu kommen, ist bereits eine relativ geringe L-Aminosäure-Überproduktion ausreichend. Denn durch Überexpression oder Abschwächung von Genen bzw. Enzymen, deren förderlicher oder schädlicher Einfluss auf die Produktion der betreffenden Aminosäure bekannt ist, sowie gegebenenfalls durch ungerichtete Mutagenese kann ausgehend von einem solchen Ausgangsstamm die L-Aminosäure-Ausbeute entsprechend erhöht werden.

Erfindungsgemäß wurde nun überraschenderweise gefunden, dass ein alkaliphiles Bakterium, nämlich ein Bakterium der Art Corynebacterium humireducens, bereits natürlicherweise die L-Aminosäuren L-Alanin, L-Glutaminsäure und L-Valin in signifikanter Menge überproduziert.

Weiterhin konnte durch Kultivierung auf einem Medium, das AEC sowie gegebenenfalls Threonin enthält, ein C. humireducens-Stamm erhalten werden, der signifikante Mengen an L-Lysin produziert.

C. humireducens stellt damit zugleich einen geeigneten Ausgangspunkt für die Herstellung weiterer L-Aminosäure-Produktionsstämme dar. Denn durch entsprechende Umleitung des bakteriellen Stoffwechsels kann die Überproduktion der genannten L-Aminosäuren in eine Überproduktion anderer gewünschter L-Aminosäuren umfunktioniert werden.

Die natürlicherweise auftretende Überproduktion an L-Alanin ist vermutlich vor allem auf eine hoch effiziente Alanin-Dehydrogenase zurückzuführen, die in C. humireducens aufgefunden wurde. Nur für wenige weitere Corynebakterien wurden bislang Alanin-Dehydrogenasen beschrieben, für keines jedoch eine derart aktive Alanin-Dehydrogenase, deren Anwesenheit bereits zu einer Akkumulation von L-Alanin im Zellinnern des Wildtyps führt.

Die natürlicherweise auftretende Überproduktion an L-Glutamat ist vermutlich vor allem auf hoch effiziente hut-Gene ("histidine utilization"-Gene) zurückzuführen. Das hut-Cluster besteht aus den vier Genen hutU (Urocanat-Hydratase), hutl (Imidazolon-Propionase), hutH (Histidin-Ammonialyase) und hutG (Formididoylglutamase). Nur für wenige weitere Corynebakterien wurden bislang hut-Gene beschrieben, für keines jedoch derart aktive hut-Gene, deren Anwesenheit bereits zu einer Akkumulation von L-Glutamat im Zellinneren des Wildtyps führt.

Ein erster Gegenstand der vorliegenden Erfindung ist daher ein Verfahren zur Überproduktion einer L-Aminosäure, dadurch gekennzeichnet, dass in diesem Verfahren C. humireducens eingesetzt wird.

Unter "L-Aminosäure" sind erfindungsgemäß insbesondere die proteinogenen L-Aminosäuren zu verstehen.

Die L-Aminosäure ist hierbei vorzugsweise ausgewählt aus L-Alanin, L-Valin, L-Aminosäuren der Glutamat-Familie, insbesondere L-Glutamat, L-Glutamin, L-Prolin und L-Arginin, sowie L-Aminosäuren der Aspartat-Familie, insbesondere L-Aspartat, L-Asparagin, L-Methionin, L-Lysin, L-Isoleucin und L-Threonin. Die L-Aminosäure ist besonders bevorzugt ausgewählt aus L-Alanin, L-Valin, L-Glutamat, L-Methionin, L-Lysin und L-Threonin, vor allem aus L-Alanin, L-Valin, L-Glutamat und L-Lysin.

Der Stamm C. humireducens wird zum ersten Mal beschrieben durch Wu et al. (International Journal of Systematic and Evolutionary Microbiology (2011), 61, 882-887). Er wurde bei der DSMZ hinterlegt unter der Hinterlegungsnummer DSM 45392, seine 16S rRNA wurde bei EMBL hinterlegt und hat die Zugangsnummer GQ421281. Bei dem Ausgangsstamm handelt es sich um ein halotolerantes, alkaliphiles, Huminsäure reduzierendes Bakterium.

Weitere Informationen über C. humireducens sind folgenden Publikationen zu entnehmen: Wu et al. (Microb. Biotechnol. (2013), 6(2), 141-149), Lin et al. (Bioresour. Technol. (2013), 136, 302-308).

Weiterer Gegenstand der vorliegenden Erfindung ist entsprechend ebenso eine Alanin-Dehydrogenase (Ald), dadurch gekennzeichnet, dass sie eine Sequenzidentität von mindestens 85 oder 90 %, vorzugsweise mindestens 92, 94, 96 oder 98 %, vor allem von 100 %, zu der Sequenz gemäß SEQ ID NO: 72 aufweist.

Weiterer Gegenstand der vorliegenden Erfindung ist daher ebenso ein Polynukleotid, das für eine erfindungsgemäße Alanin-Dehydrogenase kodiert. Es handelt sich hierbei vorzugsweise um ein Polynukleotid, das eine Sequenzidentität von mindestens 90 oder 95 %, vor allem von 100 %, zu der Sequenz von Position 301 bis 1365 gemäß SEQ ID NO: 71 aufweist.

Weiterer Gegenstand der vorliegenden Erfindung sind daher auch die Enzyme des hut-Clusters, ausgewählt aus
a) einer Urocanat-Hydratase (hutU), dadurch gekennzeichnet, dass sie eine Sequenzidentität von mindestens 85 oder 90 %, vorzugsweise mindestens 92, 94, 96 oder 98 %, vor allem 100 %, zu der Sequenz gemäß SEQ ID NO: 190 aufweist;
b) einer Imidazolon-Propionase (hutl), dadurch gekennzeichnet, dass sie eine Sequenzidentität von mindestens 85 oder 90 %, vorzugsweise mindestens 92, 94, 96 oder 98 %, vor allem 100 %, zu der Sequenz gemäß SEQ ID NO: 192 aufweist;
c) einer Histidin-Ammonialyase (hutH), dadurch gekennzeichnet, dass sie eine Sequenzidentität von mindestens 85 oder 90 %, vorzugsweise mindestens 92, 94, 96 oder 98 %, vor allem 100 %, zu der Sequenz gemäß SEQ ID NO: 194 aufweist; und
d) einer Formididoylglutamase, dadurch gekennzeichnet, dass sie eine Sequenzidentität von mindestens 85 oder 90 %, vorzugsweise mindestens 92, 94, 96 oder 98 %, vor allem 100 %, zu der Sequenz gemäß SEQ ID NO: 196 aufweist.

Weiterer Gegenstand der vorliegenden Erfindung sind daher ebenso Polynukleotide, die für die erfindungsgemäßen Gene des hut-Clusters kodieren. Es handelt sich hierbei vorzugsweise um folgende Polynukleotide:
a) ein Polynukleotid, das für eine Uroconat-Hydratase (hutU) kodiert, und eine Sequenzidentität von mindestens 70 oder 75 %, vorzugsweise von mindestens 80 oder 85 %, besonders bevorzugt von mindestens 90 oder 95 %, vor allem von 100 %, zu der Sequenz von Position 301 bis 1983 gemäß SEQ ID NO: 189 aufweist;
b) ein Polynukleotid, das für eine Imidazolon-Propionase (hutl) kodiert, und eine Sequenzidentität von mindestens 70 oder 75 %, vorzugsweise von mindestens 80 oder 85 %, besonders bevorzugt von mindestens 90 oder 95 %, vor allem von 100 %, zu der Sequenz von Position 301 bis 1509 gemäß SEQ ID NO: 191 aufweist;
c) ein Polynukleotid, das für eine Histidin-Ammonialyase (hutH) kodiert, und eine Sequenzidentität von mindestens 70 oder 75 %, vorzugsweise von mindestens 80 oder 85 %, besonders bevorzugt von mindestens 90 oder 95 %, vor allem von 100 %, zu der Sequenz von Position 301 bis 1851 gemäß SEQ ID NO: 193 aufweist; und
d) ein Polynukleotid, das für eine Formididoylglutamase (hutG) kodiert, und eine Sequenzidentität von mindestens 70 oder 75 %, vorzugsweise von mindestens 80 oder 85 %, besonders bevorzugt von mindestens 90 oder 95 %, vor allem von 100 %, zu der Sequenz von Position 301 bis 1209 gemäß SEQ ID NO: 195 aufweist.

Weiterer Gegenstand der vorliegenden Erfindung sind ebenso Polynukleotide, die zu den erfindungsgemäßen kodierenden Polynukleotiden komplementär sind.

Weiterer Aspekt der Offenbarung sind entsprechend auch Vektoren, insbesondere Klonierungs- und Expressionsvektoren, die erfindungsgemäße Polynukleotide enthalten. Diese Vektoren können entsprechend in Mikroorganismen, insbesondere in coryneforme Bakterien, vor allem der Gattung Corynebacterium, oder Enterobacteriaceae, vor allem der Gattung Escherichia, eingebaut werden.

Ein erfindungsgemäßes Polynukleotid kann weiterhin zwecks Expression der kodierten Gene auch in das Genom von Mikroorganismen, insbesondere in das Genom von coryneformen Bakterien, insbesondere solchen der Gattung Corynebacterium, oder in das Genom von Enterobacteriaceaae, insbesondere solchen der Gattung Escherichia, eingebaut werden.

Weiterer Gegenstand der vorliegenden Erfindung sind entsprechend auch rekombinante Mikroorganismen, vorzugsweise Bakterien, insbesondere coryneforme Bakterien, vor allem solche der Gattung Corynebacterium, besonders bevorzugt der Art C. humireducens oder C. glutamicum, sowie Enterobacteriaceae, vor allem solche der Gattung Escherichia, die eine erfindungsgemäße Alanin-Dehydrogenase und/oder ein oder mehrere, vorzugsweise alle, erfindungsgemäßen Enzyme des hut-Clusters und/oder ein oder mehrere erfindungsgemäße Polynukleotide enthalten.

Ein bevorzugter Gegenstand sind hierbei rekombinante Corynebakterien, insbesondere der Art C. humireducens und der Art C. glutamicum, die eine erfindungsgemäße Alanin-Dehydrogenase und/oder ein für diese kodierendes Polynukleotid enthalten.

Weiterer bevorzugter Gegenstand sind hierbei rekombinante Corynebakterien, insbesondere der Art. C. humireducens und der Art. C. glutamicum, die mindestens ein, vorzugsweise alle, Enzym(e) des hut-Clusters und/oder für diese kodierende Polynukleotide enthalten.

Ein besonderer Gegenstand der vorliegenden Erfindung sind insbesondere auch rekombinante Mikroorganismen, vorzugsweise Bakterien, insbesondere coryneforme Bakterien, vor allem solche der Gattung Cornyebacterium mit Ausnahme der Art C. humireducens, insbesondere der Art. C. glutamicum, die eine erfindungsgemäße Alanin-Dehydrogenase und/oder ein oder mehrere, vorzugsweise alle, erfindungsgemäße Enzyme des hut-Clusters und/oder ein oder mehrere erfindungsgemäße Polynukleotide enthalten.

Unter einem "rekombinanten Mikroorganismus" bzw. "rekombinanten Bakterium" ist erfindungsgemäß ein Mikroorganismus bzw. Bakterium zu verstehen, der/das mindestens einer gentechnischen Maßnahme unterzogen wurde. Bei der gentechnischen Maßnahme kann es sich hierbei insbesondere um eine zielgerichtete oder ungerichtete Mutation, den Einbau eines wirtsfremden Gens und/oder die Überexpression oder Abschwächung eines wirtseigenen oder wirtsfremden Gens handeln. Vorzugsweise zeichnet sich ein erfindungsgemäßer rekombinanter Mikroorganismus bzw. ein erfindungsgemäßes rekombinantes Bakterium durch die Überexpression oder Abschwächung mindestens eines Gens aus. In einer besonders bevorzugten Ausführungsform zeichnet sich ein erfindungsgemäßer Mikroorganismus bzw. ein erfindungsgemäßes Bakterium hierbei durch die Überexpression der erfindungsgemäßen Alanin-Dehydrogenase bzw. des für sie kodierenden Polynukleotids aus. In einer weiteren besonders bevorzugten Ausführungsform zeichnet sich ein erfindungsgemäßer Mikroorganismus bzw. ein erfindungsgemäßes Bakterium durch die Überexpression mindestens eines erfindungsgemäßen Enzyms des hut-Clusters, insbesondere aller erfindungsgemäßer Enzyme des hut-Clusters, bzw. der entsprechenden für die Enzyme kodierenden Polynukleotide aus.

Innerhalb der Gattung Corynebacterium werden Stämme bevorzugt, die auf folgenden Arten beruhen: Corynebacterium efficiens, wie zum Beispiel der Typstamm DSM44549, Corynebacterium glutamicum, wie zum Beispiel der Typstamm ATCC13032 oder der Stamm R, Corynebacterium ammoniagenes, wie zum Beispiel der Stamm ATCC6871, Corynebacterium humireducens, wie zum Beispiel der Stamm DSM 45392, sowie Corynebacterium pekinese, wie zum Beispiel der Stamm CGMCC No. 5361.

Besonders bevorzugt sind die Arten Corynebacterium glutamicum und Corynebacterium humireducens. Sofern im Rahmen dieser Anmeldung vom Stamm Corynebacterium humireducens die Rede ist, handelt es sich vorzugsweise um den Stamm DSM 45392 oder um einen davon abgeleiteten Stamm.

Einige Vertreter der Art Corynebacterium glutamicum sind im Stand der Technik auch unter anderen Bezeichnungen bekannt. Hierzu gehören beispielsweise: Corynebacterium acetoacidophilum ATCC13870, Corynebacterium lilium DSM20137, Corynebacterium melassecola ATCC17965, Brevibacterium flavum ATCC14067, Brevibacterium lactofermentum ATCC13869 und Brevibacterium divaricatum ATCC14020. Der Begriff "Micrococcus glutamicus" für Corynebacterium glutamicum war ebenfalls gebräuchlich. Einige Vertreter der Art Corynebacterium efficiens wurden im Stand der Technik auch als Corynebacterium thermoaminogenes bezeichnet, wie zum Beispiel der Stamm FERM BP-1539.

Angaben zur taxonomischen Einordnung von Stämmen der Gruppe der coryneformen Bakterien findet man unter anderem bei Seiler (Journal of General Microbiology 129, 1433-1477 (1983)), Kinoshita (1985, Glutamic Acid Bacteria, p 115-142. In: Demain and Solomon (ed), Biology of Industrial Microorganisms. The Benjamin/Cummins Publishing Co., London, UK), Kämpfer und Kroppenstedt (Canadian Journal of Microbiology 42, 989-1005 (1996)), Liebl et al (International Journal of Systematic Bacteriology 41, 255-260 (1991)), Fudou et al (International Journal of Systematic and Evolutionary Microbiology 52, 1127-1131 (2002)) und in der US-A-5,250,434.

Stämme mit der Bezeichnung "ATCC" können von der American Type Culture Collection (Manassas, VA, USA) bezogen werden. Stämme mit der Bezeichnung "DSM" können von der Deutschen Sammlung von Mikroorganismen und Zellkulturen (DSMZ, Braunschweig, Deutschland) bezogen werden. Stämme mit der Bezeichnung "NRRL" können von der Agricultural Research Service Patent Culture Collection (ARS, Peoria, Illinois, US) bezogen werden. Stämme mit der Bezeichnung "FERM" können vom National Institute of Advanced Industrial Science and Technology (AIST Tsukuba Central 6, 1-1-1 Higashi, Tsukuba Ibaraki, Japan) bezogen werden. Stämme mit der Bezeichnung "CGMCC" können vom China General Microbiological Culture Collection Center (CGMCC, Beijing, China) bezogen werden.

Weiterer Gegenstand der vorliegenden Erfindung ist entsprechend ebenso ein Verfahren zur Überproduktion einer L-Aminosäure, dadurch gekennzeichnet, dass in diesem Verfahren eine erfindungsgemäße Alanin-Dehydrogenase und/oder mindestens ein erfindungsgemäßes Enzym des hut-Clusters, vorzugsweise alle erfindungsgemäßen Enzyme des hut-Clusters, und/oder mindestens ein erfindungsgemäßes Polynukleotid und/oder ein erfindungsgemäßer rekombinanter Mikroorganismus, vorzugsweise ein erfindungsgemäßes rekombinantes Bakterium, insbesondere ein erfindungsgemäßes rekombinantes coryneformes Bakterium, besonderes bevorzugt ein erfindungsgemäßes rekombinantes Corynebakterium, vor allem ein Corynebacterium der Art C. humireducens oder C. glutamicum, eingesetzt wird. In einer erfindungsgemäß bevorzugten Ausführungsform wird hierbei das mindestens eine erfindungsgemäße Polynukleotid bzw. das durch dieses kodierte Polypeptid in überexprimierter Form eingesetzt.

Ein bevorzugter Gegenstand der vorliegenden Erfindung ist hierbei ein Verfahren zur Überproduktion einer L-Aminosäure, dadurch gekennzeichnet, dass in diesem Verfahren eine erfindungsgemäße Alanin-Dehydrogenase und/oder mindestens ein für diese kodierendes Polynukleotid und/oder ein rekombinantes Corynebakterium, vorzugsweise der Art C. humireducens oder C. glutamicum, das eine erfindungsgemäße Alanin-Dehydrogenase und/oder mindestens ein für diese kodierendes Polynukleotid enthält, eingesetzt wird.

Weiterer bevorzugter Gegenstand der vorliegenden Erfindung ist daher ebenso ein Verfahren zur Überproduktion einer L-Aminosäure, dadurch gekennzeichnet, dass in diesem Verfahren mindestens ein erfindungsgemäßes Enzym des hut-Clusters, vorzugsweise alle erfindungsgemäßen Enzyme des hut-Clusters, und/oder mindestens ein für diese(s) Enzym(e) kodierendes Polynukleotid, vorzugsweise für alle erfindungsgemäßen Enzyme des hut-Clusters kodierende Polynukleotide, und/oder ein rekombinantes Corynebakterium, vorzugsweise der Art. C. humireducens oder C. glutamicum, das mindestens ein erfindungsgemäßes Enzym des hut-Clusters, vorzugsweise alle erfindungsgemäßen Enzyme des hut-Clusters, und/oder mindestens ein für diese(s) Enzym(e) kodierendes Polynukleotid, vorzugsweise für alle erfindungsgemäßen Enzyme des hut-Clusters kodierende Polynukleotide, enthält, eingesetzt wird.

Die erfindungsgemäß produzierte L-Aminosäure ist hierbei vorzugsweise ausgewählt aus L-Alanin, L-Valin, L-Aminosäuren der Glutamat-Familie, insbesondere L-Glutamat, L-Glutamin, L-Prolin und L-Arginin, sowie L-Aminosäuren der Aspartat-Familie, insbesondere L-Aspartat, L-Asparagin, L-Methionin, L-Lysin, L-Isoleucin und L-Threonin, besonders bevorzugt ausgewählt aus L-Alanin, L-Valin, L-Glutamat, L-Methionin, L-Lysin und L-Threonin, vor allem aus L-Alanin, L-Valin, L-Glutamat und L-Lysin.

Das in erfindungsgemäßen Produktionsverfahren eingesetzte Corynebakterium ist vorzugsweise ausgewählt aus C. humireducens und C. glutamicum.

Unter "Überproduzieren" bzw. "Überproduktion" ist erfindungsgemäß in Bezug auf die L-Aminosäure zu verstehen, dass die Mikroorganismen die L-Aminosäure über ihren eigenen Bedarf hinaus produzieren und entweder in der Zelle anreichern oder in das sie umgebende Nährmedium ausscheiden und dort akkumulieren. Vorzugsweise sind die Mikroorganismen hierbei dazu in der Lage ≥ (mindestens) 0,25 g/l, ≥ 0,5 g/l, ≥ 1,0 g/l, ≥ 1,5 g/l, ≥ 2,0 g/l, ≥ 4 g/l oder ≥ 10 g/l der betreffenden L-Aminosäure in ≤ (maximal) 120 Stunden, ≤ 96 Stunden, ≤ 48 Stunden, ≤ 36 Stunden, ≤ 24 Stunden oder ≤ 12 Stunden in der Zelle oder im Nährmedium anzureichern bzw. zu akkumulieren.

Erfindungsgemäße rekombinante Mikroorganismen, in die erfindungsgemäße Polynukleotide eingebracht wurden, besitzen in einer bevorzugten Ausführungsform bereits vor dem Einbau der erfindungsgemäßen Polynukleotide und/oder Vektoren die Fähigkeit eine L-Aminosäure überzuproduzieren. Bei den Ausgangsstämmen handelt es sich bevorzugt um Stämme, die durch Mutagenese und Selektion, durch rekombinante DNA-Techniken oder durch eine Kombination beider Methoden hergestellt wurden.

Es ist offensichtlich und bedarf keiner weiteren Erklärungen, dass man zu einem erfindungsgemäßen rekombinanten Mikroorganismus auch dadurch gelangen kann, indem man einen Wildstamm, in welchem ein erfindungsgemäßes Polynukleotid und/oder ein erfindungsgemäßer Vektor enthalten ist oder eingebaut wurde, anschließend durch geeignete weitere genetische Maßnahmen dazu veranlasst, die L-Aminosäure zu produzieren bzw. die L-Aminosäure-Produktion zu erhöhen.

Weiterer Aspekt der Offenbarung sind auch weitere Polynukleotide aus C. humireducens sowie die durch diese Polynukleotide kodierten Polypeptide. Durch Überexpression der entsprechenden Polynukleotide bzw. Polypeptide kann die Aminosäureproduktion bestimmter L-Aminosäuren positiv beeinflusst werden.

Hier genannte Mikroorganismen oder Bakterien, insbesondere Corynebakterien, vor allem erfindungsgemäße Corynebakterien der Art C. humireducens oder C. glutamicum, insbesondere erfindungsgemäße L-Valin-Überproduktionsstämme, weisen bevorzugt mindestens eines, vorzugsweise mindestens 2 oder 3, besonders bevorzugt mindestens 4 oder 5, der folgenden Merkmale auf:
a) ein überexprimiertes Polynukleotid (ilvA-Gen), das für eine Threonindehydratase (IlvA, EC 4.3.1.19), vorzugsweise für eine Threonindehydratase mit einer Sequenzidentität von mindestens 90, 95 oder 98 %, vorzugsweise 100 %, zu der Sequenz gemäß SEQ ID NO: 106, kodiert,
b) ein überexprimiertes Polynukleotid (ilvB-Gen), das für die Untereinheit einer Acetolactat-Synthase (lIvB), vorzugsweise für die Untereinheit einer Acetolactat-Synthase mit einer Sequenzidentität von mindestens 90, 95 oder 98 %, vorzugsweise 100 %, zu der Sequenz gemäß SEQ ID NO: 98, kodiert,
c) ein überexprimiertes Polynukleotid (ilvN-Gen), das für die vorzugsweise feedback-resistente Untereinheit einer Acetolactat-Synthase (IlvN, EC 4.1.3.18) kodiert,
d) ein überexprimiertes Polynukleotid (ilvC-Gen), das für eine Isomeroreduktase (IlvC, EC 1.1.1.86), vorzugsweise für eine Isomeroreduktase mit einer Sequenzidentität von mindestens 90, 95 oder 98 %, vorzugsweise 100 %, zu der Sequenz gemäß SEQ ID NO: 100, kodiert,
e) ein überexprimiertes Polynukleotid (ilvD-Gen), das für eine Dihydroxy-acid Dehydratase (IlvD, EC 4.2.1.9), vorzugsweise für eine Dihydroxy-acid Dehydratase mit einer Sequenzidentität von mindestens 90, 95 oder 98 %, vorzugsweise 100 %, zu der Sequenz gemäß SEQ ID NO: 102, kodiert,
f) ein überexprimiertes Polynukleotid (ilvE-Gen), das für eine Transaminase (IlvE, EC 2.6.1.42), vorzugsweise für eine Transaminase mit einer Sequenzidentität von mindestens 90, 95 oder 98 %, vorzugsweise 100 %, zu der Sequenz gemäß SEQ ID NO: 104, kodiert,
g) ein überexprimiertes Polynukleotid (ilvH-Gen), das für eine Acetolactat-Synthase (IlvH, EC 2.2.1.6), vorzugsweise für eine Acetolactat-Synthase mit einer Sequenzidentität von mindestens 90, 95 oder 98 %, vorzugsweise 100 %, zu der Sequenz gemäß SEQ ID NO: 122, kodiert,
h) ein abgeschwächtes Polynukleotid (thrB-Gen), das für eine Homoserinkinase (ThrB, EC 2.7.1.39), vorzugsweise für eine Homoserinkinase mit einer Sequenzidentität von mindestens 90, 95 oder 98 %, vorzugsweise 100 %, zu der Sequenz gemäß SEQ ID NO: 4, kodiert,
i) ein abgeschwächtes Polynukleotid (thrC-Gen), das für eine Threonin-Synthase (ThrC, EC 4.2.3.1), vorzugsweise für eine Threonin-Synthase mit einer Sequenzidentität von mindestens 90, 95 oder 98 %, vorzugsweise 100 %, zu der Sequenz gemäß SEQ ID NO: 108, kodiert,
j) ein überexprimiertes Polynukleotid (hom-Gen), das für eine gegebenenfalls feedback-resistente Homoserin-Dehydrogenase (Hom, EC 1.2.1.11), vorzugsweise für eine Homoserin-Dehydrogenase mit einer Sequenzidentität von mindestens 90, 95 oder 98 %, vorzugsweise 100 %, zu der Sequenz gemäß SEQ ID NO: 46, kodiert,
k) ein abgeschwächtes Polynukleotid (leuA-Gen), das für eine gegebenenfalls feedback-resistente Isopropylmalat-Synthase (LeuA, EC 2.3.3.13), vorzugsweise für eine Isopropylmalat-Synthase mit einer Sequenzidentität von mindestens 90, 95 oder 98 %, vorzugsweise 100 %, zu der Sequenz gemäß SEQ ID NO: 110, kodiert,
l) ein abgeschwächtes Polynukleotide (leuB-Gen), das für eine Isopropylmalat-Dehydrogenase (LeuB, EC 1.1.1.85), vorzugsweise für eine Isopropylmalat-Dehydrogenase mit einer Sequenzidentität von mindestens 90, 95 oder 98 %, vorzugsweise 100 %, zu der Sequenz gemäß SEQ ID NO: 112, kodiert,
m) abgeschwächte Polynukleotide (leuCD-Gene), die für die Untereinheiten einer Isopropylmalat-Isomerase (LeuCD, EC 4.2.1.33), vorzugsweise für die Untereinheiten einer Isopropylmalat-Isomerase mit Sequenzidentitäten von mindestens 90, 95 oder 98 %, vorzugsweise 100 %, zu den Sequenzen gemäß SEQ ID NO: 114 und SEQ ID NO: 116, kodieren,
n) ein überexprimiertes Polynukleotid (panB-Gen), das für eine 3-Methyl-2-Oxobutanoat-Hydroxymethyltransferase (PanB, EC 2.1.2.11), vorzugsweise für eine 3-Methyl-2-Oxobutanoat-Hydroxymethyltransferase mit einer Sequenzidentität von mindestens 90, 95 oder 98 %, vorzugsweise 100 %, zu der Sequenz gemäß SEQ ID NO: 118, kodiert,
o) ein überexprimiertes Polynukleotid (panC-Gen), das für eine Pantothenat-Synthase (PanC, EC 6.3.2.1), vorzugsweise für eine Pantothenat-Synthase mit einer Sequenzidentität von mindestens 90, 95 oder 98 %, vorzugsweise 100 %, zu der Sequenz gemäß SEQ ID NO: 120, kodiert.

Weiterer Aspekt der Offenbarung ist entsprechend auch ein Verfahren zur Überproduktion einer L-Aminosäure, insbesondere L-Valin, in welchem ein derartiger Mikroorganismus bzw. ein derartiges Bakterium eingesetzt wird.

Hier genannte Mikroorganismen oder Bakterien, insbesondere Corynebakterien, vor allem erfindungsgemäße Corynebakterien der Art C. humireducens oder C. glutamicum, insbesondere erfindungsgemäße L-Glutamat-Überproduktionsstämme, weisen bevorzugt mindestens eines, vorzugsweise mindestens zwei oder drei, besonders bevorzugt mindestens vier oder fünf, der folgenden Merkmale auf, besonders bevorzugt in Kombination mit der Überexpression mindestens eines erfindungsgemäßen hut-Gens, insbesondere in Kombination mit der Überexpression aller erfindungsgemäßer hut-Gene:
a) ein überexprimiertes Polynukleotid (gdh), das für eine Glutamat-Dehydrogenase (Gdh), vorzugsweise für eine Glutamat-Dehydrogenase mit einer Sequenzidentität von mindestens 90, 95 oder 98 %, vorzugsweise 100 %, zu der Sequenz gemäß SEQ ID NO: 124 kodiert,
b) ein überexprimiertes Polynukleotid, das für eine Glutamin-Synthetase (Glutamin-Synthetase 1), vorzugsweise für eine Glutamin-Synthetase mit einer Sequenzidentität von mindestens 90, 95 oder 98 %, vorzugsweise 100 %, zu der Sequenz gemäß SEQ ID NO: 126 kodiert,
c) ein überexprimiertes Polynukleotid, das für eine Glutamin-Synthetase (Glutamin-Synthetase 2), vorzugsweise für eine Glutamin-Synthetase mit einer Sequenzidentität von mindestens 90, 95 oder 98 %, vorzugsweise 100 %, zu der Sequenz gemäß SEQ ID NO: 128 kodiert,
d) ein überexprimiertes Polynukleotid, das für eine Glutamat-Synthase, vorzugsweise für eine Glutamat-Synthase mit einer Sequenzidentität von mindestens 90, 95 oder 98 %, vorzugsweise 100 %, zu der Sequenz gemäß SEQ ID NO: 130 kodiert,
e) ein überexprimiertes Polynukleotid, das für eine Isocitrat-Dehydrogenase, vorzugsweise für eine Isocitrat-Dehydrogenase mit einer Sequenzidentität von mindestens 90, 95 oder 98 %, vorzugsweise 100 %, zu der Sequenz gemäß SEQ ID NO: 132 kodiert,
f) ein überexprimiertes Polynukleotid, das für eine Aconitat-Hydrase, vorzugsweise für eine Aconat-Hydrase mit einer Sequenzidentität von mindestens 90, 95 oder 98 %, vorzugsweise 100 %, zu der Sequenz gemäß SEQ ID NO: 134 kodiert,
g) ein überexprimiertes Polynukleotid, das für eine Citrat-Synthase, vorzugsweise für eine Citrat-Synthase mit einer Sequenzidentität von mindestens 90, 95 oder 98 %, vorzugsweise 100 %, zu der Sequenz gemäß SEQ ID NO: 136 kodiert,
h) ein überexprimiertes Polynukleotid (pepC), das für eine Aminopeptidase C (PepC), vorzugsweise für eine Aminopeptidase C mit einer Sequenzidentität von mindestens 90, 95 oder 98 %, vorzugsweise 100 %, zu der Sequenz gemäß SEQ ID NO: 138 kodiert,
i) ein überexprimiertes Polynukleotid, das für eine Pyruvat-Dehydrogenase, vorzugsweise für eine Pyruvat-Dehydrogenase mit einer Sequenzidentität von mindestens 90, 95 oder 98 %, vorzugsweise 100 %, zu der Sequenz gemäß SEQ ID NO: 140 kodiert,
j) ein überexprimiertes Polynukleotid, das für eine Pyruvat-Kinase (Pyruvat-Kinase 1), vorzugsweise für eine Pyruvat-Kinase mit einer Sequenzidentität von mindestens 90, 95 oder 98 %, vorzugsweise 100 %, zu der Sequenz gemäß SEQ ID NO: 142 kodiert,
k) ein überexprimiertes Polynukleotid, das für eine Pyruvat-Kinase (Pyruvat-Kinase 2), vorzugsweise für eine Pyruvat-Kinase mit einer Sequenzidentität von mindestens 90, 95 oder 98 %, vorzugsweise 100 %, zu der Sequenz gemäß SEQ ID NO: 144 kodiert,
l) ein überexprimiertes Polynukleotid, das für eine Enolase, vorzugsweise für eine Enolase mit einer Sequenzidentität von mindestens 90, 95 oder 98 %, vorzugsweise 100 %, zu der Sequenz gemäß SEQ ID NO: 146 kodiert,
m) ein überexprimiertes Polynukleotid (gpmA), das für eine 2,3-Bisphosphoglyceratabhängige Phosphoglycerat-Mutase (GpmA), vorzugsweise für eine Phosphoglycerat-Mutase mit einer Sequenzidentität von mindestens 90, 95 oder 98 %, vorzugsweise 100 %, zu der Sequenz gemäß SEQ ID NO: 148 kodiert,
n) ein überexprimiertes Polynukleotid (pgk), das für eine Phosphoglycerat-Kinase (Pgk), vorzugsweise für eine Phosphoglycerat-Kinase mit einer Sequenzidentität von mindestens 90, 95 oder 98 %, vorzugsweise 100 %, zu der Sequenz gemäß SEQ ID NO: 150 kodiert,
o) ein überexprimiertes Polynukleotid, das für eine Glycerinaldehyd-3-phosphat-Dehydrogenase (Glycerin-3-phosphat-Dehydrogenase 1), vorzugsweise für eine Glycerinaldehyd-3-phosphat-Dehydrogenase mit einer Sequenzidentität von mindestens 90, 95 oder 98 %, vorzugsweise 100 %, zu der Sequenz gemäß SEQ ID NO: 152 kodiert,
p) ein überexprimiertes Polynukleotid, das für eine Glycerinaldehyd-3-phosphat-Dehydrogenase (Glycerin-3-phosphat-Dehydrogenase 2), vorzugsweise für eine Glycerinaldehyd-3-phosphat-Dehydrogenase mit einer Sequenzidentität von mindestens 90, 95 oder 98 %, vorzugsweise 100 %, zu der Sequenz gemäß SEQ ID NO: 154 kodiert,
q) ein überexprimiertes Polynukleotid (tpiA), das für eine Triosephosphat-Isomerase (TpiA), vorzugsweise für eine Triosephosphat-Isomerase mit einer Sequenzidentität von mindestens 90, 95 oder 98 %, vorzugsweise 100 %, zu der Sequenz gemäß SEQ ID NO: 156 kodiert,
r) ein überexprimiertes Polynukleotid, das für eine Fructosebisphosphataldolase, vorzugsweise für eine Fructosebisphosphataldolase mit einer Sequenzidentität von mindestens 90, 95 oder 98 %, vorzugsweise 100 %, zu der Sequenz gemäß SEQ ID NO: 158 kodiert,
s) ein überexprimiertes Polynukleotid, das für eine 1-Phosphofructokinase, vorzugsweise für eine 1-Phosphofructokinase mit einer Sequenzidentität von mindestens 90, 95 oder 98 %, vorzugsweise 100 %, zu der Sequenz gemäß SEQ ID NO: 160 kodiert,
t) ein überexprimiertes Polynukleotid, das für eine 6-Phosphofructokinase, vorzugsweise für eine 6-Phosphofructokinase mit einer Sequenzidentität von mindestens 90, 95 oder 98 %, vorzugsweise 100 %, zu der Sequenz gemäß SEQ ID NO: 162 kodiert,
u) ein überexprimiertes Polynukleotid (pgi), das für eine Glucose-6-phosphat-Isomerase, vorzugsweise für eine Glucose-6-phosphat-Isomerase mit einer Sequenzidentität von mindestens 90, 95 oder 98 %, vorzugsweise 100 %, zu der Sequenz gemäß SEQ ID NO: 6 kodiert,
v) abgeschwächte Polynukleotide (sucCD), die für die Untereinheiten einer Succinyl-CoA-Ligase (SucCD, EC 6.2.1.5), vorzugsweise für die Untereinheiten einer Succinyl-CoA-Ligase mit Sequenzidentitäten von mindestens 90, 95 oder 98 %, vorzugsweise 100 %, zu den Sequenzen gemäß SEQ ID NO: 198 bzw. SEQ ID NO: 200 kodieren.

Weiterer Aspekt der Offenbarung ist entsprechend auch ein Verfahren zur Überproduktion einer L-Aminosäure, insbesondere L-Glutamat, in welchem ein derartiger Mikroorganismus bzw. ein derartiges Bakterium eingesetzt wird.

Hier genannte Mikroorganismen oder Bakterien, insbesondere Corynebakterien, vor allem erfindungsgemäße Corynebakterien der Art C. humireducens oder C. glutamicum, insbesondere erfindungsgemäße L-Alanin-Überproduktionsstämme, weisen bevorzugt mindestens eines, vorzugsweise mindestens zwei oder drei, besonders bevorzugt mindestens vier oder fünf, der folgenden Merkmale auf, besonders bevorzugt in Kombination mit der Überexpression des erfindungsgemäßen ald-Gens:
a) ein überexprimiertes Polynukleotid (alaD), das für eine Alanin-Dehydrogenase (AlaD), vorzugsweise für eine Alanin-Dehydrogenase aus Corynebakterien kodiert,
b) ein überexprimiertes Polynukleotid (gapA), das für eine Glycerinaldehyd-3-phosphat-Dehydrogenase (GapA), vorzugsweise für eine Glycerinaldehyd-3-phosphat-Dehydrogenase aus Corynebakterien kodiert,
c) ein ausgeschaltetes oder abgeschwächtes Polynukleotid (IdhA), das für eine L-Lactat-Dehydrogenase (LdhA), vorzugsweise für eine L-Lactat-Dehydrogenase aus Corynebakterien kodiert,
d) ein ausgeschaltetes oder abgeschwächtes Polynukleotid (ppc), das für eine Phosphoenolpyruvat-Carboxylase (Ppc), vorzugsweise für eine Phosphoenolpyruvat-Carboxylase aus Corynebakterien kodiert,
e) ein ausgeschaltetes oder abgeschwächtes Polynukleotid (alr), das für eine Alanin-Racemase (Alr), vorzugsweise für eine Alanin-Racemase aus Corynebakterien kodiert.

Weiterer Aspekt der Offenbarung ist entsprechend auch ein Verfahren zur Überproduktion einer L-Aminosäure, insbesondere L-Alanin, in welchem ein derartiger Mikroorganismus bzw. ein derartiges Bakterium eingesetzt wird.

Hier genannte Mikroorganismen oder Bakterien, insbesondere Corynebakterien, vor allem erfindungsgemäße Corynebakterien der Art C. humireducens oder C. glutamicum, insbesondere erfindungsgemäße L-Methionin-Überproduktionsstämme, weisen bevorzugt mindestens eines, vorzugsweise mindestens zwei oder drei, besonders bevorzugt mindestens vier oder fünf, der folgenden Merkmale auf:
a) ein abgeschwächtes Polynukleotid (mcbR), das für eine DNA-Bindungsdomäne vom Typ HTH tetR (McbR), vorzugsweise für eine DNA-Bindungsdomäne mit einer Sequenzidentität von mindestens 90, 95 oder 98 %, vorzugsweise 100 %, zu der Sequenz gemäß SEQ ID NO: 2 kodiert,
b) ein abgeschwächtes Polynukleotid (thrB-Gen), das für eine Homoserinkinase (ThrB, EC 2.7.1.39), vorzugsweise für eine Homoserinkinase mit einer Sequenzidentität von mindestens 90, 95 oder 98 %, vorzugsweise 100 %, zu der Sequenz gemäß SEQ ID NO: 4, kodiert,
c) ein abgeschwächtes Polynukleotid (pgi), das für eine Glucose-6-phosphat-Isomerase (Pgi, EC 5.3.1.9), vorzugsweise für eine Glucose-6-phosphat-Isomerase mit einer Sequenzidentität von mindestens 90, 95 oder 98 %, vorzugsweise 100 %, zu der Sequenz gemäß SEQ ID NO: 6, kodiert,
d) ein abgeschwächtes Polynukleotid (pck), das für eine Phosphoenolpyruvat-Carboxykinase (Pck, EC 4.1.1.32), vorzugsweise für eine Phosphoenolpyruvat-Carboxykinase mit einer Sequenzidentität von mindestens 90, 95 oder 98 %, vorzugsweise 100 %, zu der Sequenz gemäß SEQ ID NO: 8, kodiert,
e) ein abgeschwächtes Polynukleotid (metQ), das für ein D-Methionin bindendes Lipoprotein (MetQ), vorzugsweise für ein D-Methionin bindendes Lipoprotein mit einer Sequenzidentität von mindestens 90, 95 oder 98 %, vorzugsweise 100 %, zu der Sequenz gemäß SEQ ID NO: 10, kodiert,
f) ein abgeschwächtes Polynukleotid (metP), das für einen Methionin-Transporter (MetP), vorzugsweise für einen Methionin-Transporter mit einer Sequenzidentität von mindestens 90, 95 oder 98 %, vorzugsweise 100 %, zu der Sequenz gemäß SEQ ID NO: 12, kodiert,
g) ein abgeschwächtes Polynukleotid (metN), das für einen ATP-abhängigen Methionin-Transporter (MetN), vorzugsweise für einen ATP-abhängigen Methionin-Transporter mit einer Sequenzidentität von mindestens 90, 95 oder 98 %, vorzugsweise 100 %, zu der Sequenz gemäß SEQ ID NO: 14, kodiert,
h) ein abgeschwächtes Polynukleotid (metK), das für eine S-Adenosylmethionin-Synthase (MetK), vorzugsweise für eine S-Adenosylmethionin-Synthase mit einer Sequenzidentität von mindestens 90, 95 oder 98 %, vorzugsweise 100 %, zu der Sequenz gemäß SEQ ID NO: 16, kodiert,
i) ein abgeschwächtes Polynukleotid (metl), das für eine Methionin-Importsystem-Permease (Metl), vorzugsweise für eine Methionin-Importsystem-Permease mit einer Sequenzidentität von mindestens 90, 95 oder 98 %, vorzugsweise 100 %, zu der Sequenz gemäß SEQ ID NO: 18, kodiert,
j) ein abgeschwächtes Polynukleotid (dapA), das für eine 4-Hydroxy-Tetrahydrodipicolinat-Synthase (DapA), vorzugsweise für eine 4-Hydroxy-Tetrahydrodipicolinat-Synthase mit einer Sequenzidentität von mindestens 90, 95 oder 98 %, vorzugsweise 100 %, zu der Sequenz gemäß SEQ ID NO: 20, kodiert,
k) ein überexprimiertes Polynukleotid (CBS, cysK), das für eine Cystein-Synthase (CBS, CysK), vorzugsweise für eine Cystein-Synthase mit einer Sequenzidentität von mindestens 90, 95 oder 98 %, vorzugsweise 100 %, zu der Sequenz gemäß SEQ ID NO: 22 kodiert,
l) ein abgeschwächtes Polynukleotid, das für eine Carboxylat-Amin-Ligase, vorzugsweise für eine Carboxylat-Amin-Ligase mit einer Sequenzidentität von mindestens 90, 95 oder 98 %, vorzugsweise 100 %, zu der Sequenz gemäß SEQ ID NO: 24 kodiert,
m) ein überexprimiertes Polynukleotid (aecD), das für eine Cystathionin-Betalyase (AecD), vorzugsweise für eine Cystathionin-Betalyase mit einer Sequenzidentität von mindestens 90, 95 oder 98 %, vorzugsweise 100 %, zu der Sequenz gemäß SEQ ID NO: 26 kodiert,
n) ein überexprimiertes Polynukleotid (asd), das für eine Aspartat-semialdehyd-Dehydrogenase (Asd), vorzugsweise für eine Aspartat-semialdehyd-Dehydrogenase mit einer Sequenzidentität von mindestens 90, 95 oder 98 %, vorzugsweise 100 %, zu der Sequenz gemäß SEQ ID NO: 28 kodiert,
o) ein überexprimiertes Polynukleotid (metH), das für eine 5-Methyltetrahydrofolat-Homocysteinmethyltransferase (MetH, EC 2.1.1.13) kodiert,
p) ein überexprimiertes Polynukleotid (brnE), das für die kleinere Untereinheit eines Transporters für verzweigt-kettige Aminosäuren (BrnE), vorzugsweise für eine Untereinheit mit einer Sequenzidentität von mindestens 90, 95 oder 98 %, vorzugsweise 100 %, zu der Sequenz gemäß SEQ ID NO: 30 kodiert,
q) ein überexprimiertes Polynukleotid (brnF), das für die größere Untereinheit eines Transporters für verzweigt-kettige Aminosäuren (BrnF), vorzugsweise für eine Untereinheit mit einer Sequenzidentität von mindestens 90, 95 oder 98 %, vorzugsweise 100 %, zu der Sequenz gemäß SEQ ID NO: 32 kodiert,
r) ein überexprimiertes Polynukleotid (cysE), das für eine Serin-Acetyltransferase (CysE), vorzugsweise für eine Serin-Acetyltransferase mit einer Sequenzidentität von mindestens 90, 95 oder 98 %, vorzugsweise 100 %, zu der Sequenz gemäß SEQ ID NO: 34 kodiert,
s) ein überexprimiertes Polynukleotid (cysK), das für eine Cystein-Synthase (CysK), vorzugsweise für eine Cystein-Synthase mit einer Sequenzidentität von mindestens 90, 95 oder 98 %, vorzugsweise 100 %, zu der Sequenz gemäß SEQ ID NO: 36 kodiert,
t) ein überexprimiertes Polynukleotid (gcvH), das für das H-Protein eines Glycin spaltenden Systems (GcvH), vorzugsweise für ein H-Protein mit einer Sequenzidentität von mindestens 90, 95 oder 98 %, vorzugsweise 100 %, zu der Sequenz gemäß SEQ ID NO: 38 kodiert,
u) ein überexprimiertes Polynukleotid (gcvP), das für das P-Protein eines Glycin spaltenden Systems (GcvP), vorzugsweise für ein P-Protein mit einer Sequenzidentität von mindestens 90, 95 oder 98 %, vorzugsweise 100 %, zu der Sequenz gemäß SEQ ID NO: 40 kodiert,
v) ein überexprimiertes Polynukleotid (gcvT), das für das T-Protein eines Glycin spaltenden Systems (GcvT), vorzugsweise für ein T-Protein mit einer Sequenzidentität von mindestens 90, 95 oder 98 %, vorzugsweise 100 %, zu der Sequenz gemäß SEQ ID NO: 42 kodiert,
w) ein überexprimiertes Polynukleotid (glyA), das für eine Serin-Hydroxymethyltransferase (GlyA), vorzugsweise für eine Serin-Hydroxymethyltransferase mit einer Sequenzidentität von mindestens 90, 95 oder 98 %, vorzugsweise 100 %, zu der Sequenz gemäß SEQ ID NO: 44 kodiert,
x) ein überexprimiertes Polynukleotid (hom), das für eine gegebenenfalls feedback-resistente Homoserin-Dehydrogenase (Hom), vorzugsweise für eine Homoserin-Dehydrogenase mit einer Sequenzidentität von mindestens 90, 95 oder 98 %, vorzugsweise 100 %, zu der Sequenz gemäß SEQ ID NO: 46 kodiert,
y) ein überexprimiertes Polynukleotid (lipA), das für eine Lipoyl-Synthase (LipA), vorzugsweise für eine Lipoyl-Synthase mit einer Sequenzidentität von mindestens 90, 95 oder 98 %, vorzugsweise 100 %, zu der Sequenz gemäß SEQ ID NO: 48 kodiert,
z) ein überexprimiertes Polynukleotid (lipB), das für eine Lipoyl-Transferase (LipB), vorzugsweise für eine Lipoyl-Transferase mit einer Sequenzidentität von mindestens 90, 95 oder 98 %, vorzugsweise 100 %, zu der Sequenz gemäß SEQ ID NO: 50 kodiert,
aa) ein überexprimiertes Polynukleotid (Ipd), das für eine Dihydrolipoyl-Dehydrogenase (Lpd), vorzugsweise für eine Dihydrolipoyl-Dehydrogenase mit einer Sequenzidentität von mindestens 90, 95 oder 98 %, vorzugsweise 100 %, zu der Sequenz gemäß SEQ ID NO: 52 kodiert,
bb) ein überexprimiertes Polynukleotid (IplA), das für eine Lipoat-Protein-Ligase (LplA), vorzugsweise für eine Lipoat-Protein-Ligase mit einer Sequenzidentität von mindestens 90, 95 oder 98 %, vorzugsweise 100 %, zu der Sequenz gemäß SEQ ID NO: 94 kodiert,
cc) ein überexprimiertes Polynukleotid (gcvL), das für eine Dihydrolipoyl-Dehydrogenase (GcvL), vorzugsweise für eine Dihydrolipoyl-Dehydrogenase mit einer Sequenzidentität von mindestens 90, 95 oder 98 %, vorzugsweise 100 %, zu der Sequenz gemäß SEQ ID NO: 96, kodiert,
dd) ein überexprimiertes Polynukleotid (lysC), das für eine vorzugsweise feedback-resistente Aspartat-Kinase (LysC), vorzugsweise für eine Aspartat-Kinase mit einer Sequenzidentität von mindestens 90, 95 oder 98 %, vorzugsweise 100 %, zu der Sequenz gemäß SEQ ID NO: 54 kodiert,
ee) ein überexprimiertes Polynukleotid (metB), das für eine Cystathionin-gamma-Synthase (MetB), vorzugsweise für eine Cystathinonin-gamma-Synthase mit einer Sequenzidentität von mindestens 90, 95 oder 98 %, vorzugsweise 100 %, zu der Sequenz gemäß SEQ ID NO: 56, kodiert,
ff) ein überexprimiertes Polynukleotid (metF), das für eine 5,10-Methylentetrahydrofolat-Reduktase (MetF), vorzugsweise für eine 5,10-Methylentetrahydrofolat-Reduktase mit einer Sequenzidentität von mindestens 90, 95 oder 98 %, vorzugsweise 100 %, zu der Sequenz gemäß SEQ ID NO: 58, kodiert,
gg) ein überexprimiertes Polynukleotid (metX), das für eine Homoserin-O-Acetyltransferase (MetX), vorzugsweise für eine Homoserin-O-Acetyltransferase mit einer Sequenzidentität von mindestens 90, 95 oder 98 %, vorzugsweise 100 %, zu der Sequenz gemäß SEQ ID NO: 60, kodiert,
hh) ein überexprimiertes Polynukleotid (metY), das für eine O-Acetylhomoserin-Lyase (MetY), vorzugsweise für eine O-Acetylhomoserin-Lyase mit einer Sequenzidentität von mindestens 90, 95 oder 98 %, vorzugsweise 100 %, zu der Sequenz gemäß SEQ ID NO: 62, kodiert,
ii) ein überexprimiertes Polynukleotid (pyc), das für eine Pyruvat-Carboxylase (Pyc), vorzugsweise für eine Pyruvat-Carboxylase mit einer Sequenzidentität von mindestens 90, 95 oder 98 %, vorzugsweise 100 %, zu der Sequenz gemäß SEQ ID NO: 64, kodiert,
jj) ein überexprimiertes Polynukleotid (serA), das für eine gegebenenfalls feedback-resistente D-3-Phosphoglycerat-Dehydrogenase (SerA), vorzugsweise für eine D-3-Phosphoglycerat-Dehydrogenase mit einer Sequenzidentität von mindestens 90, 95 oder 98 %, vorzugsweise 100 %, zu der Sequenz gemäß SEQ ID NO: 66, kodiert,
kk) ein überexprimiertes Polynukleotid (serB), das für eine Phosphoserin-Phosphatase (SerB), vorzugsweise für eine Phosphoserin-Phosphatase mit einer Sequenzidentität von mindestens 90, 95 oder 98 %, vorzugsweise 100 %, zu der Sequenz gemäß SEQ ID NO: 68, kodiert,
ll) ein überexprimiertes Polynukleotid (serC), das für eine Phosphoserin-Aminotransferase (SerC), vorzugsweise für eine Phosphoserin-Aminotransferase mit einer Sequenzidentität von mindestens 90, 95 oder 98 %, vorzugsweise 100 %, zu der Sequenz gemäß SEQ ID NO: 70, kodiert,
mm) ein überexprimiertes Polynukleotid (cysD), das für die Untereinheit einer Sulfat-Adenylyltransferase (CysD), vorzugsweise für eine Untereinheit mit einer Sequenzidentität von mindestens 90, 95 oder 98 %, vorzugsweise 100 %, zu der Sequenz gemäß SEQ ID NO: 74 kodiert,
nn) ein überexprimiertes Polynukleotid (cysH), das für eine Adenosin-Phosphosulfat-Reduktase (CysH), vorzugsweise für eine Adenosin-Phosphosulfat-Reduktase mit einer Sequenzidentität von mindestens 90, 95 oder 98 %, vorzugsweise 100 %, zu der Sequenz gemäß SEQ ID NO: 76 kodiert,
oo) ein überexprimiertes Polynukleotid (cysl), das für eine Sulfit-Reduktase (Cysl), vorzugsweise für eine Sulfit-Reduktase mit einer Sequenzidentität von mindestens 90, 95 oder 98 %, vorzugsweise 100 %, zu der Sequenz gemäß SEQ ID NO: 78 kodiert,
pp) ein überexprimiertes Polynukleotid (cysJ), das für (CysJ), vorzugsweise für eine mit einer Sequenzidentität von mindestens 90, 95 oder 98 %, vorzugsweise 100 %, zu der Sequenz gemäß SEQ ID NO: 80, kodiert,
qq) ein überexprimiertes Polynukleotid (cysN), das für die Untereinheit einer Sulfat-Adenylyltransferase (CysD), vorzugsweise für eine Untereinheit mit einer Sequenzidentität von mindestens 90, 95 oder 98 %, vorzugsweise 100 %, zu der Sequenz gemäß SEQ ID NO: 82, kodiert,
rr) ein überexprimiertes Polynukleotid (cysY), das für eine Cystathionin-beta-Synthase (CysY), vorzugsweise für eine Cystathionin-beta-Synthase mit einer Sequenzidentität von mindestens 90, 95 oder 98 %, vorzugsweise 100 %, zu der Sequenz gemäß SEQ ID NO: 84, kodiert,
ss) ein überexprimiertes Polynukleotid (cysZ), das für einen putativen Sulfat-Transporter (CysZ), vorzugsweise für einen Sulfat-Transporter mit einer Sequenzidentität von mindestens 90, 95 oder 98 %, vorzugsweise 100 %, zu der Sequenz gemäß SEQ ID NO: 86, kodiert,
tt) ein überexprimiertes Polynukleotid (metE), das für eine 5-Methyltetrahydropteroyltriglutamat-homocystein-Methyltransferase (MetE), vorzugsweise für ein Protein mit einer Sequenzidentität von mindestens 90, 95 oder 98 %, vorzugsweise 100 %, zu der Sequenz gemäß SEQ ID NO: 88, kodiert,
uu) ein überexprimiertes Polynukleotid (ptH1), das für eine Peptidyl-tRNA-Hydrolase 1 (PtH1), vorzugsweise für eine Peptidyl-tRNA-Hydrolase 1 mit einer Sequenzidentität von mindestens 90, 95 oder 98 %, vorzugsweise 100 %, zu der Sequenz gemäß SEQ ID NO: 90, kodiert,
vv) ein überexprimiertes Polynukleotid (ptH2), das für eine Peptidyl-tRNA-Hydrolase 2 (PtH2), vorzugsweise für eine eine Peptidyl-tRNA-Hydrolase 2 mit einer Sequenzidentität von mindestens 90, 95 oder 98 %, vorzugsweise 100 %, zu der Sequenz gemäß SEQ ID NO: 92, kodiert.

Weiterer Aspekt der Offenbarung ist entsprechend auch ein Verfahren zur Überproduktion einer L-Aminosäure, insbesondere L-Methionin, in welchem ein derartiger Mikroorganismus bzw. ein derartiges Bakterium eingesetzt wird.

Hier genannte Mikroorganismen oder Bakterien, insbesondere Corynebakterien, vor allem erfindungsgemäße Corynebakterien der Art C. humireducens oder C. glutamicum, insbesondere erfindungsgemäße L-Lysin-Überproduktionsstämme, weisen bevorzugt mindestens eines, vorzugsweise mindestens 2 oder 3, besonders bevorzugt mindestens 4 oder 5, der folgenden Merkmale auf:
a) ein überexprimiertes Polynukleotid (dapA), das für eine Dihydrodipicolinat-Synthase (DapA, EC 4.2.1.52), vorzugsweise für eine Dihydrodipicolinat-Synthase mit einer Sequenzidentität von mindestens 90, 95 oder 98 %, vorzugsweise 100 %, zu der Sequenz gemäß SEQ ID NO: 20, kodiert,
b) ein überexprimiertes Polynukleotid (lysC), das für eine vorzugsweise feedback-resistente Aspartat-Kinase (LysC, EC 2.7.2.4), vorzugsweise für eine Aspartat-Kinase mit einer Sequenzidentität von mindestens 90, 95 oder 98 %, vorzugsweise 100 %, zu der Sequenz gemäß SEQ ID NO: 54 kodiert,
c) ein überexprimiertes Polynukleotid (ddh), das für eine Diaminopimelat-Dehydrogenase (Ddh, EC 1.4.1.16), vorzugsweise für eine Diaminopimelat-Dehydrogenase mit einer Sequenzidentität von mindestens 90, 95 oder 98 %, vorzugsweise 100 %, zu der Sequenz gemäß SEQ ID NO: 202 kodiert,
d) ein überexprimiertes Polynukleotid (asd), das für eine Aspartat-Semialdehyd-Dehydrogenase (Asd, EC 1.2.1.11), vorzugsweise für eine eine Aspartat-Semialdehyd-Dehydrogenase mit einer Sequenzidentität von mindestens 90, 95 oder 98 %, vorzugsweise 100 %, zu der Sequenz gemäß SEQ ID NO: 28, kodiert,
e) ein überexprimiertes Polynukleotid (lysA), das für eine Diaminopimelat-Decarboxylase (LysA, EC 4.1.1.20), vorzugsweise für eine Diaminopimelat-Decarboxylase mit einer Sequenzidentität von mindestens 90, 95 oder 98 %, vorzugsweise 100 %, zu der Sequenz gemäß SEQ ID NO: 164, kodiert,
f) ein überexprimiertes Polynukleotid (aat), das für eine Aspartat-Aminotransferase (AaT, EC 2.6.1.1), vorzugsweise für eine Aspartat-Aminotransferase mit einer Sequenzidentität von mindestens 90, 95 oder 98 %, vorzugsweise 100 %, zu der Sequenz gemäß SEQ ID NO: 166, kodiert,
g) ein überexprimiertes Polynukleotid (lysE), das für einen L-Lysin-Exporter (LysE, Lysin-Efflux-Permease), vorzugsweise für einen L-Lysin-Exporter mit einer Sequenzidentität von mindestens 90, 95 oder 98 %, vorzugsweise 100 %, zu der Sequenz gemäß SEQ ID NO: 168, kodiert,
h) ein überexprimiertes Polynukleotid (pyc), das für eine Pyruvat-Carboxylase (Pyc, EC 6.4.1.1), vorzugsweise für eine Pyruvat-Carboxylase mit einer Sequenzidentität von mindestens 90, 95 oder 98 %, vorzugsweise 100 %, zu der Sequenz gemäß SEQ ID NO: 64, kodiert,
i) ein überexprimiertes Polynukleotid (dapF), das für eine Diaminopimelat-Epimerase (DapF, EC 5.1.1.7) kodiert,
j) ein überexprimiertes Polynukleotid (dapB), das für eine Dihydropicolinat-Reduktase (DapB, EC 1.3.1.26), vorzugsweise für eine Dihydropicolinat-Reduktase mit einer Sequenzidentität von mindestens 90, 95 oder 98 %, vorzugsweise 100 %, zu der Sequenz gemäß SEQ ID NO: 170, kodiert,
k) ein überexprimiertes Polynukleotid, das für eine Glucose-6-phosphat-Dehydrogenase (EC 1.1.1.49), vorzugsweise für eine Glucose-6-phosphat-Dehydrogenase mit einer Sequenzidentität von mindestens 90, 95 oder 98 %, vorzugsweise 100 %, zu der Sequenz gemäß SEQ ID NO: 172, kodiert,
l) ein überexprimiertes Polynukleotid (zwf), das für die Zwf-Untereinheit einer Glucose-6-phosphat-Dehydrogenase (Zwf, EC 1.1.1.49), vorzugsweise für eine Zwf-Untereinheit mit einer Sequenzidentität von mindestens 90, 95 oder 98 %, vorzugsweise 100 %, zu der Sequenz gemäß SEQ ID NO: 186, kodiert,
m) ein überexprimiertes Polynukleotid (opcA), das für die OpcA-Untereinheit einer Glucose-6-phosphat-Dehydrogenase (OpcA, EC 1.1.1.49), vorzugsweise für eine OpcA-Untereinheit mit einer Sequenzidentität von mindestens 90, 95 oder 98 %, vorzugsweise 100 %, zu der Sequenz gemäß SEQ ID NO: 188, kodiert,
n) ein überexprimiertes Polynukleotid (gnd), das für eine Phosphogluconsäure-Dehydrogenase (Gnd, EC 1.1.1.44), vorzugsweise für eine Phosphogluconsäure-Dehydrogenase mit einer Sequenzidentität von mindestens 90, 95 oder 98 %, vorzugsweise 100 %, zu der Sequenz gemäß SEQ ID NO: 174, kodiert,
o) ein ausgeschaltetes oder abgeschwächtes Polynukleotid (mqo), das für eine Malat-Chinon-Oxidoreduktase (Mqo, EC 1.1.99.16), vorzugsweise für eine Malat-Chinon-Oxidoreduktase mit einer Sequenzidentität von mindestens 90, 95 oder 98 %, vorzugsweise 100 %, zu der Sequenz gemäß SEQ ID NO: 176, kodiert,
p) ein ausgeschaltetes oder abgeschwächtes Polynukleotid, das für die E1p-Untereinheit eines Pyruvat-Dehydrogenase-Komplexes (AceE, EC 1.2.4.1), vorzugsweise für eine E1p-Untereinheit mit einer Sequenzidentität von mindestens 90, 95 oder 98 %, vorzugsweise 100 %, zu der Sequenz gemäß SEQ ID NO: 178, kodiert,
q) ein ausgeschaltetes oder abgeschwächtes Polynukleotid (gltA), das für eine Citrat-Synthase (GltA, EC 4.1.3.7), vorzugsweise für eine Citrat-Synthase mit einer Sequenzidentität von mindestens 90, 95 oder 98 %, vorzugsweise 100 %, zu der Sequenz gemäß SEQ ID NO: 180, kodiert,
r) ein ausgeschaltetes oder abgeschwächtes Polynukleotid (mdh), das für eine Malat-Dehydrogenase (Mdh, EC 1.1.1.37), vorzugsweise für eine Malat-Dehydrogenase mit einer Sequenzidentität von mindestens 90, 95 oder 98 %, vorzugsweise 100 %, zu der Sequenz gemäß SEQ ID NO: 182, kodiert,
s) ein ausgeschaltetes oder abgeschwächtes Polynukleotid (murE), das für eine UDP-N-acetylmuramoylalanyl-D-glutamat-2,6-diaminopimelat-Ligase, 6-Diaminopimelat-Ligase (MurE, EC 6.3.2.13), vorzugsweise für ein Enzym mit einer Sequenzidentität von mindestens 90, 95 oder 98 %, vorzugsweise 100 %, zu der Sequenz gemäß SEQ ID NO: 184, kodiert.

Weiterer Aspekt der Offenbarung ist entsprechend auch ein Verfahren zur Überproduktion einer L-Aminosäure, insbesondere L-Lysin, in welchem ein derartiger Mikroorganismus bzw. ein derartiges Bakterium eingesetzt wird.

Die zuvor genannten, in erfindungsgemäßen Verfahren eingesetzten bzw. einzusetzenden Polynukleotide und Polypeptide stammen vorzugsweise aus Corynebakterien, insbesondere aus C. glutamicum oder C. humireducens, besonders bevorzugt aus C. humireducens.

Unter "Überexpression" ist erfindungsgemäß allgemein eine Erhöhung der intrazellulären Konzentration oder Aktivität einer Ribonukleinsäure, eines Proteins (Polypeptids) oder eines Enzyms, die durch eine entsprechende DNA kodiert werden, in einem Mikroorganismus im Vergleich zum Ausgangsstamm (Elternstamm) oder Wildtypstamm zu verstehen. Unter einem Ausgangsstamm (Elternstamm) versteht man den Stamm, an dem die zur Überexpression führende Maßnahme durchgeführt wurde.

Die Erhöhung der Konzentration oder Aktivität lässt sich beispielsweise dadurch erzielen, dass man die Kopienzahl der entsprechenden kodierenden Polynukleotide chromosomal oder extrachromosomal um mindestens eine Kopie erhöht.

Eine weit verbreitete Methode zur Erhöhung der Kopienzahl besteht darin, dass man das entsprechende kodierende Polynukleotid in einen Vektor, bevorzugt ein Plasmid, einbaut, der von einem Mikroorganismuns, insbesondere einem coryneformen Bakterium, repliziert wird. Weiterhin kann man als Vektoren Transposons, Insertionselemente (IS-Elemente) oder Phagen einsetzen. Im Stand der Technik ist eine Fülle geeigneter Vektoren beschrieben.

Eine andere verbreitete Methode zur Erzielung einer Überexpression ist das Verfahren der chromosomalen Genamplifikation. Bei dieser Methode wird mindestens eine zusätzliche Kopie des interessierenden Polynukleotids in das Chromosom eines coryneformen Bakteriums eingefügt. Derartige Amplifikationsverfahren sind beispielsweise in der WO 03/014330 oder WO 03/040373 beschrieben.

Eine weitere Methode zur Erzielung einer Überexpression besteht darin, das entsprechende Gen beziehungsweise Allel in funktioneller Weise (operably linked) mit einem Promotor beziehungsweise einer Expressionskassette zu verknüpfen. Geeignete Promotoren für Corynebacterium glutamicum sind beispielsweise in der Fig. 1 des Übersichtsartikel von Patek et al. (Journal of Biotechnology 104(1-3), 311-323 (2003)) und in zusammenfassenden Darstellungen wie dem "Handbook of Corynebacterium glutamicum" (Eds.: Lothar Eggeling und Michael Bott, CRC Press, Boca Raton, US (2005)) oder dem Buch "Corynebacteria, Genomics and Molecular Biology" (Ed.: Andreas Burkovski, Caister Academic Press, Norfolk, UK (2008)) beschrieben. In gleicher Weise können die von Vasicova et al (Journal of Bacteriology 181, 6188-6191 (1999)) beschriebenen Varianten des dapA-Promotors, beispielsweise der Promotor A25 eingesetzt werden. Weiterhin kann der gap-Promotor von Corynebacterium glutamicum (EP 06007373) verwendet werden. Schließlich können die hinlänglich bekannten von Amann et al. (Gene 69(2), 301-315 (1988)) und Amann und Brosius (Gene 40(2-3), 183-190 (1985)) beschriebenen Promotoren T3, T7, SP6, M13, lac, tac und trc verwendet werden. Ein derartiger Promotor kann beispielsweise stromaufwärts des betreffenden Gens, typischerweise im Abstand von ungefähr 1 - 500 Nukleobasen vom Startkodon, eingefügt werden.

Durch die Maßnahme der Überexpression wird die Aktivität oder Konzentration des entsprechenden Polypeptids vorzugsweise um mindestens 10%, 25%, 50%, 75%, 100%, 150%, 200%, 300%, 400% oder 500%, maximal bis vorzugsweise 1000% oder 2000%, bezogen auf die Aktivität oder Konzentration des Polypeptids im Stamm vor der zur Überexpression führenden Maßnahme, erhöht.

Die Konzentration eines Proteins kann über 1- und 2-dimensionale Proteingelauftrennung und anschließende optische Identifizierung der Proteinkonzentration mit entsprechender Auswertesoftware im Gel bestimmt werden. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit entsprechender Software zur Konzentrationsbestimmung (Lohaus und Meyer (1998) Biospektrum 5:32-39; Lottspeich, Angewandte Chemie 38: 2630-2647 (1999)) bestimmt werden. Die Aktivität kann mit Hilfe eines geeigneten Enzymtest bestimmt werden.

Der Begriff "Abschwächung" bezeichnet erfindungsgemäß die Verringerung der intrazellulären Konzentration oder Aktivität einer Ribonukleinsäure, eines Proteins (Polypeptids) oder eines Enzyms, die durch eine entsprechende DNA kodiert werden, in einem Mikroorganismus, im Vergleich zum Ausgangsstamm (Elternstamm) oder Wildtypstamm. Als Ausgangsstamm (Elternstamm) wird der Stamm bezeichnet, an dem die Maßnahme der Abschwächung durchgeführt wurde.

Die Abschwächung kann erzielt werden, indem die Expression eines Polypeptids, beispielsweise durch Verwendung eines schwachen Promotors, verringert wird oder indem ein Allel verwendet wird, das für ein Polypeptid mit einer niedrigeren Aktivität kodiert und gegebenenfalls diese Maßnahmen kombiniert. Die Abschwächung kann auch dadurch erreicht werden, indem die Expression des Polypeptids vollständig unterbunden wird, beispielsweise dadurch, dass das kodierende Gen ausgeschaltet wird.

Durch die Maßnahme der Abschwächung wird die Aktivität oder Konzentration des entsprechenden Polypeptids vorzugsweise um mindestens 10%, 25%, 50% oder 75%, maximal um 100%, bezogen auf die Aktivität oder Konzentration des Polypeptids im Stamm vor der zur Abschwächung führenden Maßnahme, reduziert. Bevorzugt besteht die Abschwächung in der vollständigen Ausschaltung der Expression des betreffenden Polypeptids.

Unter feedback-resistenten Enzymen versteht man im Zusammenhang mit der Aminosäureproduktion generell Enzyme, die im Vergleich zur Wildform eine geringere Empfindlichkeit gegenüber der Hemmung durch die produzierte L-Aminosäure und/oder Analoga davon aufweisen.

So versteht man insbesondere unter einer feedback-resistenten Aspartatkinase (LysC^{FBR}) eine Aspartatkinase, die im Vergleich zur Wildform eine geringere Empfindlichkeit gegenüber der Hemmung durch Mischungen von Lysin und Threonin oder Mischungen von AEC (Aminoethylcystein) und Threonin oder Lysin alleine oder AEC alleine aufweisen. Für die Lysin-Produktion werden entsprechend Stämme, die solche feedbackresistenten bzw. desensibilisierten Aspartatkinasen enthalten, bevorzugt eingesetzt.

Literaturbekannt sind beispielsweise folgende feedback-resistenten Aspartatkinasen aus C. glutamicum: A279T, A279V, S301F, S301Y, T308I, T311I, R320G, G345D, S381F. Bzgl. feedback-resistenten Aspartatkinasen aus C. glutamicum wird weiterhin auf folgende Veröffentlichungen verwiesen: JP1993184366-A, JP1994062866-A, JP1994261766-A, JP1997070291-A, JP1997322774-A, JP1998165180-A, JP1998215883-A, US5688671-A, EP0387527, WO00/63388, US3732144, JP6261766, Jetten et al. (1995; Applied Microbiology Biotechnology 43: 76-82). Feedback-resistente Aspartatkinasen aus C. glutamicum sind in der NCBI-GenBank unter folgenden Zugangsnummern hinterlegt: E05108, E06825, E06826, E06827, E08177, E08178, E08179, E08180, E08181, E08182, E12770, E14514, E16352, E16745, E16746, I74588, I74589, I74590, I74591, I74592, I74593, I74594, I74595, I74596, I74597, X57226, L16848, L27125.

Bevorzugt werden folgende feedback-resistenten Aspartatkinasen aus C. humireducens eingesetzt: D274Y, A279E, S301Y, T308I, T311I, G359D.

Ebenso werden bei der Threonin-Produktion bevorzugt Stämme eingesetzt, die entsprechend eine feedback-resistente Homoserin-Dehydrogenase (Hom^{FBR}) enthalten.

Ebenso werden bei der Isoleucin-Produktion und Valin-Produktion bevorzugt Stämme eingesetzt, die entsprechend eine feedback-resistente Acetolactat-Synthase enthalten.

Ebenso werden bei der Leucin-Produktion bevorzugt Stämme eingesetzt, die entsprechend eine feedback-resistente Isopropylmalat-Synthase (LeuA^{FBR}) enthalten.

Ebenso werden bei der Prolin-Produktion bevorzugt Stämme eingesetzt, die entsprechend eine feedback-resistente Glutamat-5-Kinase (ProB^{FBR}) enthalten.

Ebenso werden bei der Arginin-Produktion bevorzugt Stämme eingesetzt, die entsprechend eine feedback-resistente Ornithin-Carbamoyltransferase (ArgF^{FBR}) enthalten.

Ebenso werden bei der Serin-Produktion bevorzugt Stämme eingesetzt, die entsprechend eine feedback-resistente D-3-Phosphoglycerat-Dehydrogenase (SerA^{FBR}) enthalten.

Ebenso werden bei der Methionin-Produktion bevorzugt Stämme eingesetzt, die entsprechend eine feedback-resistente D-3-Phosphoglycerat-Dehydrogenase (SerA^{FBR}) und/oder feedback-resistente Pyruvat-Carboxylasen (Pyc^{FBR}) enthalten.

Ebenso werden bei der Tryptophan-Produktion bevorzugt Stämme eingesetzt, die entsprechend eine feedback-resistente Phospho-2-dehydro-3-Deoxyheptonat-Aldolase (AroG^{FBR} oder AroH^{FBR}) enthalten.

Hinsichtlich weiterer bevorzugter Eigenschaften des erfindungsgemäß einzusetzenden L-Aminosäuren überproduzierenden C. humireducens-Stamms wird auf die oben zitierte Veröffentlichung von Wu et al. (2011) sowie die weiteren oben genannten Veröffentlichungen verwiesen.

Erfindungsgemäße Mikroorganismen, insbesondere Bakterien der Gattung Corynebacterium, können kontinuierlich - wie beispielsweise in der WO 05/021772 beschrieben- oder diskontinuierlich im Batch-Verfahren (Satzkultivierung bzw. Satzverfahren) oder im Fed-Batch- (Zulaufverfahren) oder Repeated-Fed-Batch Verfahren (repetitives Zulaufverfahren) zum Zwecke der Produktion der L-Aminosäure kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Als Kohlenstoffquelle können Zucker und Kohlenhydrate wie z.B. Glucose, Saccharose, Lactose, Fructose, Maltose, Melasse, Saccharose-haltige Lösungen aus der Zuckerrüben- oder Zuckerrohrherstellung, Stärke, Stärkehydrolysat und Cellulose, Öle und Fette, wie zum Beispiel Sojaöl, Sonnenblumenöl, Erdnussöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure oder Milchsäure verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muss weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden.

Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete, selektiv wirkende Stoffe, wie zum Beispiel Antibiotika hinzugefügt werden. Um aerobe Bedingungen aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Druck von 0,03 bis 0,2 MPa, gefahren. Die Temperatur der Kultur liegt normalerweise bei 20°C bis 45°C und vorzugsweise bei 25°C bis 40°C. Bei batch-Verfahren wird die Kultivierung solange fortgesetzt, bis sich ein Maximum der gewünschten L-Aminosäure, gebildet hat. Dieses Ziel wird normalerweise innerhalb von 10 Stunden bis 160 Stunden erreicht. Bei kontinuierlichen Verfahren sind längere Kultivierungszeiten möglich. Durch die Tätigkeit der Bakterien kommt es zu einer Anreicherung (Akkumulation) der L-Aminosäure im Fermentationsmedium und/oder in den Bakterienzellen.

Beispiele für geeignete Fermentationsmedien finden sich unter anderem in den Patentschriften 5,770,409, US 5,840,551 und US 5,990,350 oder US 5,275,940.

Die Analyse von L-Aminosäuren zur Bestimmung der Konzentration zu einem oder mehreren Zeitpunkt(en) im Verlauf der Fermentation kann durch Trennung der L-Aminosäuren mittels lonenaustauschchromatographie vorzugsweise Kationenaustauschchromatographie mit anschließender Nachsäulenderivatisierung unter Verwendung von Ninhydrin erfolgen, so wie bei Spackman et al. (Analytical Chemistry 30: 1190-1206 (1958)) beschrieben. Anstelle von Ninhydrin kann auch ortho-Phtadialdehyd zur Nachsäulenderivatisierung eingesetzt werden. Einen Übersichtsartikel zur lonenaustauschchromatographie findet man bei Pickering (LC·GC (Magazine of Chromatographic Science) 7(6), 484-487 (1989)).

Es ist ebenfalls möglich eine Vorsäulenderivatisierung beispielsweise unter Verwendung von ortho-Phtadialdehyd oder Phenylisothiocyanat vorzunehmen und die entstandenen Aminosäurederivate durch Reversed-Phase-Chromatographie (RP) vorzugsweise in Form der Hochleistungsflüssigkeits-chromatographie (HPLC) aufzutrennen. Eine derartige Methode ist beispielsweise bei Lindroth et al. (Analytical Chemistry 51: 1167-1174 (1979)) beschrieben.

Die Detektion erfolgt photometrisch (Absorption, Fluoreszenz).

Eine zusammenfassende Darstellung zur Aminosäureanalyse findet man unter anderem im Lehrbuch "Bioanalytik" von Lottspeich und Zorbas (Spektrum Akademischer Verlag, Heidelberg, Deutschland 1998).

Ein besonderer Aspekt der Offenbarung ist auch ein Verfahren zur Herstellung einer L-Aminosäure, dadurch gekennzeichnet, dass man folgende Schritte durchführt:
a) Fermentation der erfindungsgemäßen Mikroorganismen, insbesondere coryneformen Bakterien, bevorzugt der Gattung Corynebacterium, besonders bevorzugt der Art Corynebacterium glutamicum oder Corynebacterium humireducens, in einem geeignetem Nährmedium, und
b) Akkumulation der L-Aminosäure in dem Nährmedium und/oder in den Zellen der genannten Bakterien.

Anschließend erfolgt die Bereitstellung bzw. Herstellung oder Gewinnung eines L-Aminosäure haltigen Produktes in flüssiger oder fester Form.

Durch die Maßnahmen der Fermentation erhält man eine Fermentationsbrühe, welche die betreffende L-Aminosäure enthält.

Unter einer Fermentationsbrühe versteht man ein Fermentationsmedium bzw. Nährmedium, in dem ein Mikroorganismus für eine gewisse Zeit und bei einer gewissen Temperatur kultiviert wurde. Das Fermentationsmedium beziehungsweise die während der Fermentation eingesetzten Medien enthält/enthalten sämtliche Substanzen beziehungsweise Komponenten, die eine Vermehrung des Mikroorganismus und eine Bildung der gewünschten L-Aminosäure sicherstellen.

Bei Abschluss der Fermentation enthält die entstandene Fermentationsbrühe dementsprechend
a) die infolge der Vermehrung der Zellen des Mikroorganismus entstandene Biomasse (Zellmasse) des Mikroorganismus,
b) das im Laufe der Fermentation gebildete L-Aminosäure,
c) die im Laufe der Fermentation gebildeten organischen Nebenprodukte, und
d) die durch die Fermentation nicht verbrauchten Bestandteile des eingesetzten Fermentationsmediums beziehungsweise der Einsatzstoffe wie beispielsweise Vitamine wie Biotin oder Salze wie Magnesiumsulfat.

Zu den organischen Nebenprodukten gehören Stoffe, die von den bei der Fermentation eingesetzten Mikroorganismen neben der gewünschten L-Aminosäure erzeugt und gegebenenfalls ausgeschieden werden. Hierzu gehören auch Zucker wie zum Beispiel Trehalose.

Die Fermentationsbrühe wird dem Kulturgefäß beziehungsweise dem Fermentationsbehälter entnommen, gegebenenfalls gesammelt, und dazu verwendet, ein L-Aminosäure haltiges Produkt in flüssiger oder fester Form bereitzustellen. Hierfür wird auch der Ausdruck "Gewinnen des L-Aminosäure haltigen Produktes" verwendet. Im einfachsten Fall stellt die L-Aminosäure-haltige Fermentationsbrühe selbst das gewonnene Produkt dar.

Durch eine oder mehrere der Maßnahmen ausgewählt aus der Gruppe
a) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%) Entfernung des Wassers,
b) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%) Entfernung der Biomasse, wobei diese gegebenenfalls vor der Entfernung inaktiviert wird,
c) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%, ≥ 99,3%, ≥ 99,7%) Entfernung der im Laufe der Fermentation gebildeten organischen Nebenprodukte, und
d) teilweise (> 0%) bis vollständige (100%) oder nahezu vollständige (≥ 80%, ≥ 90%, ≥ 95%, ≥ 96%, ≥ 97%, ≥ 98%, ≥ 99%, ≥ 99,3%, ≥ 99,7%) Entfernung der durch die Fermentation nicht verbrauchten Bestandteile des eingesetzten Fermentationsmediums beziehungsweise der Einsatzstoffe,
aus der Fermentationsbrühe erzielt man eine Konzentrierung bzw. Reinigung der L-Aminosäure. Auf diese Weise werden Produkte isoliert, die einen gewünschten Gehalt an L-Aminosäure aufweisen.

Die teilweise (> 0% bis < 80%) bis vollständige (100%) oder nahezu vollständige (≥ 80% bis < 100%) Entfernung des Wassers (Maßnahme a)) wird auch als Trocknung bezeichnet.

Durch vollständige oder nahezu vollständige Entfernung des Wassers, der Biomasse, der organischen Nebenprodukte und der nicht verbrauchten Bestandteile des eingesetzten Fermentationsmediums gelangt man zu reinen ((≥ 80 Gew.-%, ≥ 90 Gew.-%) oder hochreinen (≥ 95 Gew.-%, ≥ 97 Gew.-%, ≥ 99% Gew.-%) Produktformen der L-Aminosäure. Für die Maßnahmen gemäß a), b), c) oder d) ist im Stand der Technik eine Fülle von technischen Anleitungen verfügbar.

### Ausführungsbeispiele

### Beispiel 1: L-Alanin- und L-Valin-Leistungstest

Für den L-Alanin-/L-Valin-Leistungstest wurde der Typstamm von C. *humireducens* (DSM 45392) im Schüttelkolbenansatz kultiviert. Dazu wurde der C. *humireducens* Stamm in 10 ml BHI-Flüssigmedium (Brain-Heart-Infusion; Merck) (37 g/l H₂O) als Vorkultur bei 37°C mit 200 rpm 24 h lang inkubiert. Anschließend wurden 10 ml Schüttelkolbenmedium auf eine OD₆₆₀ von 0,2 inokuliert und bei 37°C mit 200 rpm, 48 h lang kultiviert. Zur Herstellung dieses Mediums wurden 20 g Ammoniumsulfat, 0,4 g MgSO₄^{∗}7H₂O, 0,6 g KH₂PO₄ und 10 g Hefeextrakt in 750 ml H₂O gelöst. Der pH-Wert der Lösung wurde mit 20% NH₄OH auf 7,8 eingestellt und die Lösung anschließend autoklaviert. Anschließend wurden 4 ml einer Vitaminlösung (pH 7 mit NH₄OH), bestehend aus 0,25 g/l Thiamin, 50 mg/l Cyanocobalamin, 25 mg/l Biotin und 1,25 g/l Pyridoxin, hinzugefügt. Des Weiteren wurden 140 ml einer sterilfiltrierten 50%igen Glukoselösung und 50 g trockenautoklaviertes CaCO₃ hinzugefügt und das Medium anschließend auf einen Liter aufgefüllt.

Nach der Kultivierung wurde jeweils vom Überstand von vier parallelen Kulturen eine HPLC-Analyse zur Bestimmung des Gehaltes an Alanin und Valin mit einer Nachweisgrenze von ≥0,01 g/l durchgeführt.

Der Typstamm von C. *humireducens* produziert nach 48 h Kultivierung in Schüttelkolbenmedium bei 37°C, 200 rpm im Schüttelkolbenmaßstab rund 0,81 g/l Alanin (Netto-Ausbeute: 0,011 g_{Alanin}/g_{Glukose}) und 1,6 g/l Valin (Netto-Ausbeute: 0,022 gᵥₐₗᵢₙ/g_{Glukose}) (Tab. 1).

**Tab. 1: Analytikwerte eines Schüttelkolbenversuches mit dem Typstamm von C.humireducens. Aufgeführt sind die gemessenen Werte nach der Kultivierung mit Zellen und die des leeren Mediums.**

| | **Alanin (g/l)** | **Valin (g/l)** |
|---|---|---|
| | | |
| **C. humireducens** | 1,27 | 1,9 |
| | | |
| **Leer-Medium ohne Zellen** | 0,46 | 0,3 |

### Beispiel 2: Glutamat-Leistungstest

Für den L-Glutamat-Leistungstest wurde der Typstamm von C. *humireducens* (DSM 45392) im Schüttelkolbenansatz kultiviert. Dazu wurde der C. *humireducens* Stamm in 10 ml BHI-Flüssigmedium (Brain-Heart-Infusion; Merck) (37 g/l H₂O) als Vorkultur bei 37°C mit 200 rpm 24 h lang inkubiert. Anschließend wurden 10 ml Schüttelkolbenmedium auf eine OD₆₆₀ von 0,2 inokuliert und bei 37°C mit 200 rpm, 48 h lang kultiviert. Zur Herstellung dieses Mediums wurden 20 g Ammoniumsulfat, 0,4 g MgSO₄^{∗}7H₂O, 0,6 g KH₂PO₄ und 10 g Hefeextrakt in 750 ml H₂O gelöst. Der pH-Wert der Lösung wurde mit 20% NH₄OH auf 7,8 eingestellt und die Lösung anschließend autoklaviert. Anschließend wurden 4 ml einer Vitaminlösung (pH 7 mit NH₄OH), bestehend aus 0,25 g/l Thiamin, 50 mg/l Cyanocobalamin, 25 mg/l Biotin und 1,25 g/l Pyridoxin hinzugefügt. Des Weiteren wurden 140 ml einer sterilfiltrierten 50%igen Glukoselösung und 50 g trockenautoklaviertes CaCO₃ hinzugefügt. Daraufhin kamen noch 5 ml einer 400 mM sterilfiltierten Threonin-Stammlösung hinzu und das Medium wurde anschließend auf einen Liter aufgefüllt.
Nach der Kultivierung wurde jeweils vom Überstand von vier parallelen Kulturen eine HPLC-Analyse zur Bestimmung des Glutamatgehaltes mit einer Nachweisgrenze von ≥0,01 g/l durchgeführt.

Der Typstamm von C. *humireducens* produzierte nach 48 h Kultivierung in Schüttelkolbenmedium bei 37°C, 200 rpm im Schüttelkolbenmaßstab 1,8 (+/-0,6) g/l L-Glutamat. Die Ausgangskonzentration an L- Glutamat im Medium lag bei 0,78 (+/-0,1) g/l.

### Beispiel 4: AEC-Screening

Zehn Einzelklone des wildtypischen Stammes C. *humireducens* (DSM 45392) wurden in jeweils 10 ml BHI-Flüssigmedium (Brain-Heart-Infusion; Merck) (37 g/l H₂O) in Schüttelkolben über Nacht bei 37°C mit 200 rpm kultiviert. Anschließend wurden jeweils 100 µl der Übernachtkultur auf Minimalmedium-Agarplatten mit 25 mM S-2-Aminoethyl-L-Cystein (AEC) (MW=164 g/mol) ausplattiert und drei Tage lang bei 37°C inkubiert. Zur Herstellung des Minimalmediums wurden 5 g (NH₄)₂SO₄, 5 g Harnstoff, 2 g KH₂PO₄, 2 g K₂HPO₄, 10 g MOPS in 750 ml H₂O gelöst, der pH-Wert wurde mit 1 M KOH auf 7,6 eingestellt und autoklaviert. Die restlichen Bestandteile wurden getrennt angesetzt und steril filtriert. Dabei wurden 20 ml 50% (w/v) Glukose, 1 ml 1% (w/v) CaCl₂, 1 ml 1 M MgSO₄, 1 ml 0,02% Biotin und 1 ml Spurenelementlösung (1 g FeSO₄ x 7 H₂O, 1 g MnSO₄ 7 x H₂O, 0,1 g ZnSO₄ x 7 H₂O, 0,021 g CuSO₄ x 5 H₂O, 0,002 g NiCl₂ x 6 H₂O auf 100 ml H₂O) dem Medium hinzugefügt und anschließend mit sterilem H₂O auf 1000 ml aufgefüllt. Für die Kultivierung auf Festmediumplatten wurde dem Medium 15 g/l Agar-Agar (Merck) zugesetzt. Sichtbare Einzelkolonien wurden erneut auf frische Minimalmedium-Agarplatten mit 25 mM AEC und 12,5 mM Threonin als fraktionierter Ausstrich ausplattiert und drei Tage bei 37°C inkubiert. Daraufhin wurden jeweils 10 ml BHI-Flüssigmedium (Brain-Heart-Infusion; Merck) (37 g/l H₂O) im Schüttelkolben mit einem Einzelklon inokuliert und über Nacht bei 37°C mit 200 rpm schüttelnd inkubiert, anschließend mit 10% Glycerin versetzt und bei -80°C als Rückstellmuster gelagert.

### Sequenzanalyse des lysC-Sequenz Bereiches

Zur Analyse der *lysC*-Sequenz Bereiche der isolierten Einzelklone, wurde der entsprechende Genbereich von *lysC* mittels Primern (lysC_for: 5'AGACGAAAGGCGGCCTACAC3' und lysC_rev: 5TCCAGGATCGAGCGCATCAC3') und PCR-Technik amplifiziert. Die erhaltenen DNA-Sequenzen wurden mit Hilfe der Software Clone Manager analysiert. Durch die Analyse der *lysC*-Sequenz der isolierten AEC + Threonin resistenten C. *humireducens* Klone wurden folgende Punktmutationen identifiziert:

**Tab. 2: Identifizierte Veränderungen im lysC-Gen von AEC + Threonin resistenten C. humireducens Klonen und dadurch verursachte Aminosäureaustausche.**

| ***C. humireducens* Klone** | **Punktmutation** | **AS-Austausch** |
|---|---|---|
| | | |
| C. humireducens_AEC_Thr_r#1 | C → T | T308I |
| C. humireducens_AEC_Thr_r#2 | G → A | G359D |
| C. humireducens_AEC_Thr_r#3 | C → T | T311I |
| C. humireducens_AEC_Thr_r#4 | C → T | T311I |
| C. humireducens_AEC_Thr_r#5 | G → T | D274Y |
| C. humireducens_AEC_Thr_r#6 | C → T | T308I |
| C. humireducens_AEC_Thr_r#7 | C → A | S301Y |
| C. humireducens_AEC_Thr_r#9 | C → T | T308I |
| C. humireducens_AEC_Thr_r#10 | C → A | A279E |

### Beispiel 5: L-Lysin-Leistungstest

Der Typstamm *C. humireducens* und die isolierten Einzelklone aus dem AEC + Threonin Screening wurden in Schüttelkolben kultiviert und einem Leistungstest bezüglich ihrer Lysinsynthese im Schüttelkolbenmaßstab unterzogen. Hierzu wurde der *C. humireducens* Stamm und die isolierten AEC + Threonin resistenten *C. humireducens* Klone in 10 ml BHI-Flüssigmedium (Brain-Heart-Infusion; Merck) (37 g/l H₂O) als Vorkultur bei 37°C mit 200 rpm 24 h lang inkubiert. Anschließend wurden 10 ml Schüttelkolbenmedium auf eine OD₆₆₀ von 0,2 inokuliert und bei 37°C mit 200 rpm, 48 h lang kultiviert. Zur Herstellung dieses Mediums wurden 7,5 g Cornsteep Liquor (50%), 20 g Morpholinopropansulfonsäure (MOPS), 25 g (NH₄)₂SO₄, 0,1 g KH₂PO₄, 1 g MgSO₄^{∗}7H₂O, 0,01 g CaCl₂^{∗}2H₂O, 0,01 g FeSO₄^{∗}7H₂O, 0,005 g MnSO₄^{∗}H₂O in 750 ml H₂O gelöst, der pH-Wert wurde mit Ammoniakwasser auf 7,0 eingestellt und autoklaviert. Anschließend wurden 25 g trocken autoklaviertes CaCO₃ hinzugefügt. Die restlichen Bestandteile wurden getrennt angesetzt und steril filtriert. Dabei wurden 90 ml 50% (w/v) Glukose und 10 ml einer Lösung von 30 mg/l Thiamin und 20 mg/l Biotin dem Medium hinzugefügt und anschließend mit sterilem H₂O auf 1000 ml aufgefüllt.

Nach der Kultivierung wurde jeweils vom Überstand zwei paralleler Kulturen eine HPLC-Analyse zur Bestimmung der L-Lysingehalte mit einer Nachweisgrenze von ≥0,01 g/l durchgeführt. Die Lysin-Endtiter und Ausbeuten der Kultivierungen sind in der nachfolgenden Tabelle dargestellt.

**Tab. 3: Mittelwerte und Standardabweichung der Lysin-Endtiter von zwei parallelen Kulturen mit AEC + Threonin resistenten C. humireducens Klonen nach 48 h Kultivierung in Schüttelkolbenmedium bei 37°C, 200 rpm.**

| | Lysin (g/l) | |
|---|---|---|
| | Mittelwert | Stabw. |
| Typstamm C. humireducens | 0,13 | 0,01 |
| C. humireducens_AEC_Thr_r#1 | 1,33 | 0,08 |
| C. humireducens_AEC_Thr_r#2 | 1,33 | 0,08 |
| C. humireducens_AEC_Thr_r#3 | 1,25 | 0,01 |
| C. humireducens_AEC_Thr_r#4 | 1,29 | 0,01 |
| C. humireducens_AEC_Thr_r#5 | 1,24 | 0,09 |
| C. humireducens_AEC_Thr_r#6 | 0,85 | 0,04 |
| C. humireducens_AEC_Thr_r#7 | 1,12 | 0,00 |
| C. humireducens_AEC_Thr_r#9 | 1,18 | 0,02 |
| C. humireducens_AEC_Thr_r#10 | 1,34 | 0,03 |

## Patentansprüche

1. Verfahren zur Überproduktion einer L-Aminosäure, **dadurch gekennzeichnet, dass** in diesem Verfahren Corynebacterium humireducens eingesetzt wird.

2. Alanin-Dehydrogenase, **dadurch gekennzeichnet, dass** sie eine Sequenzidentität von mindestens 85 % zu der Sequenz gemäß SEQ ID NO: 72 aufweist.

3. Polynukleotid, das für eine Alanin-Dehydrogenase kodiert, ausgewählt aus der Gruppe bestehend aus:
a) Polynukleotide, die für eine Alanin-Dehydrogenase gemäß Anspruch 2 kodieren;
b) Polynukleotide, die eine Sequenzidentität von mindestens 90 % zu der Sequenz von Position 301 bis 1365 gemäß SEQ ID NO: 71 aufweisen;
c) Polynukleotide, die zu Polynukleotiden gemäß (a) oder (b) komplementär sind.

4. Enzyme des hut-Clusters, ausgewählt aus
a) einer Urocanat-Hydratase (hutU), **dadurch gekennzeichnet, dass** sie eine Sequenzidentität von mindestens 85 % zu der Sequenz gemäß SEQ ID NO: 190 aufweist;
b) einer Imidazolon-Propionase (hutl), **dadurch gekennzeichnet, dass** sie eine Sequenzidentität von mindestens 85 % zu der Sequenz gemäß SEQ ID NO: 192 aufweist;
c) einer Histidin-Ammonialyase (hutH), **dadurch gekennzeichnet, dass** sie eine Sequenzidentität von mindestens 85 % zu der Sequenz gemäß SEQ ID NO: 194 aufweist; und
d) einer Formididoylglutamase, **dadurch gekennzeichnet, dass** sie eine Sequenzidentität von mindestens 85 % zu der Sequenz gemäß SEQ ID NO: 196 aufweist.

5. Polynukleotide, die für Enzyme des hut-Clusters kodieren, ausgewählt aus
a) Polynukleotid, das für eine Uroconat-Hydratase (hutU) gemäß Anspruch 4(a) kodiert und vorzugsweise eine Sequenzidentität von mindestens 70 % zu der Sequenz von Position 301 bis 1983 gemäß SEQ ID NO: 189 aufweist;
b) Polynukleotid, das für eine Imidazolon-Propionase (hutl) gemäß Anspruch 4(b) kodiert und vorzugsweise eine Sequenzidentität von mindestens 70 % zu der Sequenz von Position 301 bis 1509 gemäß SEQ ID NO: 191 aufweist;
c) Polynukleotid, das für eine Histidin-Ammonialyase (hutH) gemäß Anspruch 4(c) kodiert und vorzugsweise eine Sequenzidentität von mindestens 70 % zu der Sequenz von Position 301 bis 1851 gemäß SEQ ID NO: 193 aufweist; und
d) Polynukleotid, das für eine Formididoylglutamase (hutG) gemäß Anspruch 4(d) kodiert und vorzugsweise eine Sequenzidentität von mindestens 70 % zu der Sequenz von Position 301 bis 1209 gemäß SEQ ID NO: 195 aufweist.
e) Polynukleotide, die zu den Polynukleotiden gemäß (a) bis (d) komplementär sind.

6. Rekombinanter Mikroorganismus, **dadurch gekennzeichnet, dass** er mindestens eine Alanin-Dehydrogenase gemäß Anspruch 2 und/oder mindestens ein Polynukleotid gemäß Anspruch 3 enthält.

7. Rekombinanter Mikroorganismus nach Anspruch 6, **dadurch gekennzeichnet, dass** in diesem Mikroorganismus die Alanin-Dehydrogenase gemäß Anspruch 2 und/oder das Polynukleotid gemäß Anspruch 3 in überexprimierter Form vorliegt.

8. Rekombinanter Mikroorganismus, **dadurch gekennzeichnet, dass** er mindestens ein Enzym, vorzugsweise alle Enzyme, des hut-Clusters gemäß Anspruch 4 und/oder mindestens ein Polynukleotid, vorzugsweise für alle Enzyme des hut-Clusters kodierende Polynukleotide, gemäß Anspruch 5 enthält.

9. Rekombinanter Mikroorganismus nach Anspruch 8, **dadurch gekennzeichnet, dass** das mindestens eine Enzym, vorzugsweise alle Enzyme, des hut-Clusters gemäß Anspruch 4 und/oder das mindestens eine Polynukleotid, vorzugsweise alle für Enzyme des hut-Clusters kodierende Polynukleotide gemäß Anspruch 5 in überexprimierter Form vorliegen.

10. Rekombinanter Mikroorganismus nach einem der Ansprüche 6 bis 9, **dadurch gekennzeichnet, dass** es sich bei dem Mikroorganismus um ein Corynebacterium, vorzugsweise der Art C. humireducens oder C. glutamicum, handelt.

11. Verfahren zur Überproduktion einer L-Aminosäure in einem Mikroorganismus, **dadurch gekennzeichnet, dass** in diesem Verfahren ein Polypeptid gemäß Anspruch 2 oder 4 und/oder ein Polynukleotid gemäß Anspruch 3 oder 5 und/oder ein rekombinanter Mikroorganismus nach einem der Ansprüche 6 bis 10 eingesetzt wird.

12. Verfahren nach einem der Ansprüche 1 oder 11, **dadurch gekennzeichnet, dass** die überproduzierte L-Aminosäure ausgewählt ist aus L-Alanin, L-Valin, L-Glutaminsäure, L-Glutamin, L-Prolin, L-Arginin, L-Asparaginsäure, L-Asparagin, L-Methionin, L-Lysin, L-Isoleucin und L-Threonin.

13. Verfahren nach Anspruch 12, **dadurch gekennzeichnet, dass** die überproduzierte L-Aminosäure ausgewählt ist aus L-Alanin, L-Valin, L-Glutaminsäure und L-Lysin.

## Claims

1. Method for the overproduction of an L-amino acid, **characterized in that** Corynebacterium humireducens is used in said method.

2. Alanine dehydrogenase, **characterized in that** said enzyme has a sequence identity of at least 85% to the sequence according to SEQ ID NO: 72.

3. Polynucleotide, which codes for an alanine dehydrogenase, selected from the group consisting of:
a) polynucleotides, which code for an alanine dehydrogenase according to Claim 2;
b) polynucleotides having a sequence identity of at least 90% to the sequence of position 301 to 1365 according to SEQ ID NO: 71;
c) polynucleotides, which are complementary to polynucleotides according to (a) or (b).

4. Enzymes of the hut cluster, selected from
a) a urocanate hydratase (hutU), **characterized in that** said enzyme has a sequence identity of at least 85% to the sequence according to SEQ ID NO: 190;
b) an imidazolonepropionase (hutI), **characterized in that** said enzyme has a sequence identity of at least 85% to the sequence according to SEQ ID NO: 192;
c) a histidine ammonia-lyase (hutH), **characterized in that** said enzyme has a sequence identity of at least 85% to the sequence according to SEQ ID NO: 194; and
d) a formimidoylglutamase, **characterized in that** said enzyme has a sequence identity of at least 85%, to the sequence according to SEQ ID NO: 196.

5. Polynucleotides, which code for enzymes of the hut cluster, selected from
a) polynucleotide, which codes for a urocanate hydratase (hutU) according to Claim 4(a) and preferably has a sequence identity of at least 70% to the sequence of position 301 to 1983 according to SEQ ID NO: 189;
b) polynucleotide, which codes for an imidazolonepropionase (hutI) according to Claim 4(b) and preferably has a sequence identity of at least 70% to the sequence of position 301 to 1509 according to SEQ ID NO: 191;
c) polynucleotide, which codes for a histidine ammonia-lyase (hutH) according to Claim 4(c) and preferably has a sequence identity of at least 70% to the sequence of position 301 to 1851 according to SEQ ID NO: 193;
d) polynucleotide, which codes for a formimidoylglutamase (hutG) according to Claim 4(d) and preferably has a sequence identity of at least 70% to the sequence of position 301 to 1209 according to SEQ ID NO: 195; and
e) polynucleotides, which are complementary to the polynucleotides according to (a) to (d).

6. Recombinant microorganism, **characterized in that** said microorganism comprises at least one alanine dehydrogenase according to Claim 2 and/or at least one polynucleotide according to Claim 3.

7. Recombinant microorganism according to Claim 6, **characterized in that** the alanine dehydrogenase according to Claim 2 and/or the polynucleotide according to Claim 3 is present in said microorganism in overexpressed form.

8. Recombinant microorganism, **characterized in that** said microorganism comprises at least one enzyme, preferably all enzymes, of the hut cluster according to Claim 4 and/or at least one polynucleotide, preferably polynucleotides coding for all enzymes of the hut cluster, according to Claim 5.

9. Recombinant microorganism according to Claim 8, **characterized in that** the at least one enzyme, preferably all enzymes, of the hut cluster according to Claim 4 and/or the at least one polynucleotide, preferably polynucleotides coding for all enzymes of the hut cluster, according to Claim 5, are present in overexpressed form.

10. Recombinant microorganism according to any of Claims 6 to 9, **characterized in that** said microorganism is a Corynebacterium, preferably of the species C. humireducens or C. glutamicum.

11. Method for the overproduction of an L-amino acid in a microorganism, **characterized in that** a polypeptide according to Claim 2 or 4 and/or a polynucleotide according to Claim 3 or 5 and/or a recombinant microorganism according to any of Claims 6 to 10 is used in said method.

12. Method according to either of Claims 1 and 11, **characterized in that** the overproduced L-amino acid is selected from L-alanine, L-valine, L-glutamic acid, L-glutamine, L-proline, L-arginine, L-aspartic acid, L-asparagine, L-methionine, L-lysine, L-isoleucine and L-threonine.

13. Method according to Claim 12, **characterized in that** the overproduced L-amino acid is selected from L-alanine, L-valine, L-glutamic acid and L-lysine.

## Revendications

1. Procédé de surproduction d'un acide L-aminé, **caractérisé en ce que** Corynebacterium humireducens est utilisé dans ce procédé.

2. Alanine déshydrogénase, **caractérisée en ce qu'**elle présente une identité de séquence d'au moins 85 % avec la séquence selon SEQ ID NO : 72.

3. Polynucléotide, qui code pour une alanine déshydrogénase, choisi dans le groupe constitué par :
a) les polynucléotides, qui codent pour une alanine déshydrogénase selon la revendication 2 ;
b) les polynucléotides, qui présentent une identité de séquence d'au moins 90 % avec la séquence de la position 301 à 1365 selon SEQ ID NO : 71 ;
c) les polynucléotides, qui sont complémentaires des polynucléotides selon (a) ou (b).

4. Enzymes du cluster hut, choisies parmi :
a) une urocanate hydratase (hutU), **caractérisée en ce qu'**elle présente une identité de séquence d'au moins 85 % avec la séquence selon SEQ ID NO : 190 ;
b) une imidazolone propionase (hutI), **caractérisée en ce qu'**elle présente une identité de séquence d'au moins 85 % avec la séquence selon SEQ ID NO : 192 ;
c) une histidine ammonialyase (hutH), **caractérisée en ce qu'**elle présente une identité de séquence d'au moins 85 % avec la séquence selon SEQ ID NO : 194 ; et
d) une formididoylglutamase, **caractérisée en ce qu'**elle présente une identité de séquence d'au moins 85 % avec la séquence selon SEQ ID NO : 196.

5. Polynucléotides, qui codent pour des enzymes du cluster hut, choisis parmi :
a) un polynucléotide qui code pour une urocanate hydratase (hutU) selon la revendication 4(a) et présente de préférence une identité de séquence d'au moins 70 % avec la séquence de la position 301 à 1983 selon SEQ ID NO : 189 ;
b) un polynucléotide qui code pour une imidazolone propionase (hutI) selon la revendication 4(b) et présente de préférence une identité de séquence d'au moins 70 % avec la séquence de la position 301 à 1509 selon SEQ ID NO : 191 ;
c) un polynucléotide qui code pour une histidine ammonialyase (hutH) selon la revendication 4(c) et présente de préférence une identité de séquence d'au moins 70 % avec la séquence de la position 301 à 1851 selon SEQ ID NO : 193 ; et
d) un polynucléotide qui code pour une formididoylglutamase (hutG) selon la revendication 4(d) et présente de préférence une identité de séquence d'au moins 70 % avec la séquence de la position 301 à 1209 selon SEQ ID NO : 195 ;
e) des polynucléotides qui sont complémentaires des polynucléotides selon (a) à (d).

6. Microorganisme recombinant, **caractérisé en ce qu'**il contient au moins une alanine déshydrogénase selon la revendication 2 et/ou au moins un polynucléotide selon la revendication 3.

7. Microorganisme recombinant selon la revendication 6, **caractérisé en ce que** l'alanine déshydrogénase selon la revendication 2 et/ou le polynucléotide selon la revendication 3 se présentent sous forme surexprimée dans ce microorganisme.

8. Microorganisme recombinant, **caractérisé en ce qu'**il contient au moins une enzyme, de préférence toutes les enzymes, du cluster hut selon la revendication 4 et/ou au moins un polynucléotide, de préférence des polynucléotides codant pour toutes les enzymes du cluster hut, selon la revendication 5.

9. Microorganisme recombinant selon la revendication 8, **caractérisé en ce que** ladite au moins une enzyme, de préférence toutes les enzymes, du cluster hut selon la revendication 4 et/ou ledit au moins un polynucléotide, de préférence tous les polynucléotides codant pour des enzymes du cluster hut, selon la revendication 5, se présentent sous forme surexprimée.

10. Microorganisme recombinant selon l'une quelconque des revendications 6 à 9, **caractérisé en ce que** le microorganisme consiste en une bactérie du genre Corynebacterium, de préférence de l'espèce C. humireducens ou C. glutamicum.

11. Procédé de surproduction d'un acide L-aminé dans un microorganisme, **caractérisé en ce qu'**un polypeptide selon la revendication 2 ou 4 et/ou un polynucléotide selon la revendication 3 ou 5 et/ou un microorganisme recombinant selon l'une quelconque des revendications 6 à 10 sont utilisés dans ce procédé.

12. Procédé selon l'une quelconque des revendications 1 ou 11, **caractérisé en ce que** l'acide L-aminé surproduit est choisi parmi la L-alanine, la L-valine, l'acide L-glutamique, la L-glutamine, la L-proline, la L-arginine, l'acide L-asparaginique, la L-asparagine, la L-méthionine, la L-lysine, la L-isoleucine et la L-thréonine.

13. Procédé selon la revendication 12, **caractérisé en ce que** l'acide L-aminé surproduit est choisi parmi la L-alanine, la L-valine, l'acide L-glutamique et la L-lysine.
